# EUROPEAN PATENT SPECIFICATION

(11) **EP 4 279 113 B1**
(45) Date of publication and mention of the grant of the patent: **13.08.2025**
(21) Application number: 23165554.9
(22) Date of filing: 30.03.2023
(51) Int. Cl.: A61M 25/00

(54) **MULTI-FILAR CATHETER BODY CONSTRUCTION**
MEHRFASERIGE KATHETERKÖRPERKONSTRUKTION
STRUCTURE DE CORPS DE CATHÉTER MULTIFILAIRE

(30) Priority: 30.03.2022 US 202263325320 P
(43) Date of publication of application: 22.11.2023
(73) Proprietor: OrbusNeich Medical Pte. Ltd., Singapore 338729 (SG)
(72) Inventor: COTTONE, Robert J., Davie, FL 33330 (US); JUMAN, Mohamad Ike, Davie, FL 33331 (US); MORALES, Jesus, Sunrise, FL 33351 (US)
(74) Representative: Hoffmann Eitle

(56) References cited:
- US-A1- 2013 331 820
- US-A1- 2014 276 117
- US-A1- 2020 038 630
- US-A1- 2020 324 080
- US-A1- 2021 338 975
- US-B2- 10 335 575

## Description

### FIELD OF THE INVENTION

The present disclosure relates to intravascular medical devices and methods of use thereof. No methods are claimed.

### BACKGROUND OF THE INVENTION

Intravascular catheters are used in a variety of different treatment and diagnostic procedures to advance and position therapeutic devices to target locations within the body. Reaching such target locations may involve navigating tortuous vascular pathways having extreme turning or bending radiuses and/or dimensions making access difficult for typical catheters. While there are a large number of vascular catheters and microcatheters designed to enable access to different anatomical locations in the vasculature, a key issue that small diameter catheter designs face is providing a combination of desirable pushability, fine torqueability response, axial strength stability, maintaining lumen concentricity, resistance to catheter body plastic deformation or catheter body fatigue and desired variable flexibility across the length of the catheter. Providing and controlling such characteristics is important in order to enable a physician to negotiate access through various complex and often tortuous, anatomical vasculature in the cardiovascular, peripheral vascular, or neurovascular systems to deliver other adjunctive devices, imaging contrasts, aspirate, and other therapeutic agents.

There remains a need for improved designs for such catheters, and more generally, alternative designs for catheter tubes and substructures that allow not only ease of fabrication, but also design control of various characteristics of the components of a catheter, including steerability, torqueability, variable bending flexibility along the working length, pushability, lumen collapse or kink resistance, axial pull strength, tip to catheter body transition compliance, etc., at any point along the catheter.

US 2020/324080 A discloses a catheter with a zone comprising a cut pattern segment defining a plurality of spiral cuts with a width of 0.1 mm, a pitch of 0.5 mm and a zone diameter of approximately 2 mm. US 2021/338975 A discloses a catheter with a spiral cut.

### SUMMARY OF THE INVENTION

The present disclosure provides a catheter as described in claim 1, including a catheter tube defining: a proximal end; a distal end; a longitudinal axis; a lumen therethrough; and a first zone having a first cut pattern segment therein. The first cut pattern segment defines a plurality of spiral cuts spaced around a circumference of the tube, where each of the plurality of spiral cuts defines a width between 0.015 mm and 0.040 mm, where each of the plurality of spiral cuts is spaced from an adjacent cut by a filar having a width between 0.015 mm and 0.125 mm, and where each of the plurality of spiral cuts defines a pitch angle with the longitudinal axis between 30 degrees and 70 degrees. The first zone may have a stiffness between approximately 0 gF/mm and approximately 0.75 gF/mm. The plurality of spiral cuts may include between 10 and 40 cuts. The first zone may have a length between 1 mm and 100 mm. The catheter tube may be constructed from a nitinol and may have a wall thickness between approximately 0.0254 mm and approximately 0.254 mm. The catheter tube may be constructed from an inconel and may have a wall thickness between approximately 0.0254 mm and approximately 0.254 mm. The catheter tube may be constructed from stainless steel and may have a wall thickness between approximately 0.0254 mm and approximately 0.254 mm.

The distal end may include a tip segment having a plurality of helical segments extending around the longitudinal axis. Each helical segment may include two substantially parallel filars connected at distal ends thereof. The plurality of helical segments may include at least 2 helical segments.

The catheter may include a polymer liner disposed within the lumen and/or a polymer jacket disposed around the outer diameter of the cut tube.

The catheter tube may include a second zone having a second cut pattern segment therein, where the second cut pattern segment defines a plurality of cuts spaced around a circumference of the tube, where each of the plurality of cuts defines a width between 0.015 mm and 0.04 mm, where each of the plurality of cuts is spaced from an adjacent cut by a filar having a width between 0.015 mm and 0.125 mm, and where each of the plurality of cuts defines a pitch angle with the longitudinal axis between 45 degrees and 55 degrees.

The second zone may be located proximal of the first zone and/or have a stiffness between approximately 0.75 gF/mm and approximately 1.25 gF/mm. The plurality of cuts of the second cut pattern segment may include between 1 and 40 cuts. The second zone may have a length between 1 mm and 100 mm.

### BRIEF DESCRIPTION OF THE DRAWINGS

A more complete understanding of the present disclosure, and the attendant advantages and features thereof, will be more readily understood by reference to the following detailed description when considered in conjunction with the accompanying drawings wherein:
FIG. 1 is an example of a medical device constructed in accordance with the principles of this disclosure;
FIG. 2 is an example of a distal portion of a catheter body constructed in accordance with the principles of this disclosure;
FIG. 3 is a closer view of a portion of a catheter body tube constructed in accordance with the principles of this disclosure;
FIG. 4 is another closer view of a portion of a catheter body constructed in accordance with the principles of this disclosure;
FIG. 5 is an example of a portion of a catheter body having alternating pitch directions constructed in accordance with the principles of this disclosure;
FIGS. 6A-D are examples of cut tube portions of a catheter body constructed in accordance with the principles of this disclosure;
FIGS. 6E-F are examples of flat cut tube cut pattern portions of a catheter body constructed in accordance with the principles of this disclosure;
FIGS. 6G are examples of cut tube portions of a catheter body constructed in accordance with the principles of this disclosure;
FIG. 6H are examples of cut tube portions of a catheter body constructed in accordance with the principles of this disclosure;
FIG. 7 is an example of a distal cut tube segment in a catheter distal tip section using cross-sectional rendering of tip construction of a medical device constructed in accordance with the principles of this disclosure;
FIG. 8 is a perspective view of the distal tip segment of FIG. 7;
FIG. 9 is another example of a cut tube component of a distal tip segment of a medical device constructed in accordance with the principles of this disclosure;
FIG. 10 is a perspective view of another example of a cut tube component of a distal tip segment of a medical device constructed in accordance with the principles of this disclosure;
FIG. 11 is a perspective view of another example of a cut tube section of another example of a distal tip segment of a medical device constructed in accordance with the principles of this disclosure;
FIG. 12 is a perspective view of another example of a cut tube section of another example of a distal tip segment of a medical device constructed in accordance with the principles of this disclosure;
FIGS. 13A-G are additional examples of cut tube sections of various distal tip segment configurations of a medical device constructed in accordance with the principles of this disclosure;
FIG. 14 is a graph showing 3-point bend test performance of examples of a catheter body constructed in accordance with the principles of this disclosure;
FIG. 15 is a graph showing 3-point bend test performance of examples of a catheter body with polymer layers constructed in accordance with the principles of this disclosure;
FIG. 16 is a graph showing frictional force test performance of examples of a catheter body constructed in accordance with the principles of this disclosure;
FIG. 17 is a graph showing axial tensile test performance of an example of a catheter body constructed in accordance with the principles of this disclosure; and
FIG. 18. is a graph showing torque shaft transmission test performance of an example of a catheter body constructed in accordance with the principles of this disclosure.

### DETAILED DESCRIPTION OF THE INVENTION

The present disclosure provides improved designs for catheter constructions, and more generally, alternative designs for catheter tubes and substructures that allow not only ease of fabrication, but also control and improvement of various characteristics of the components definable along the length of a catheter, providing a combination of desirable pushability, including steerability, fine torqueability response, axial pull strength, maintaining lumen stability and concentricity, using variable bending stiffness and flexibility along the catheter working length, circumferential collapse or kink bend resistance, resistance to catheter body plastic deformation or catheter body fatigue, etc., at any point along the catheter.

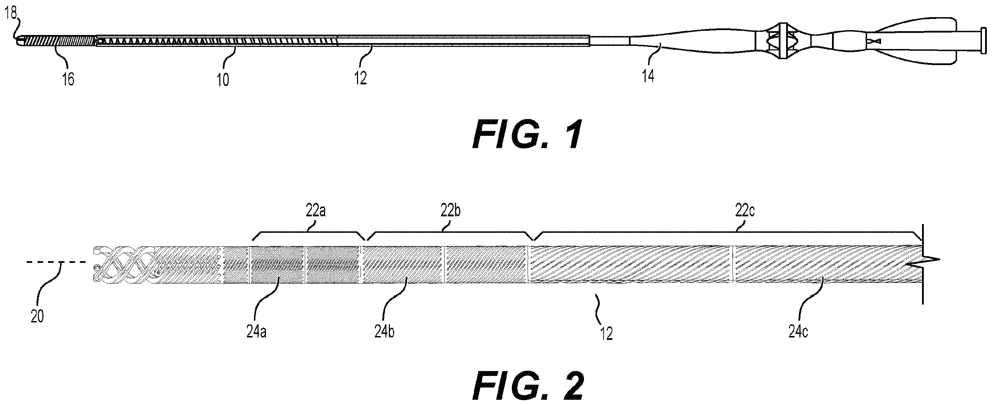

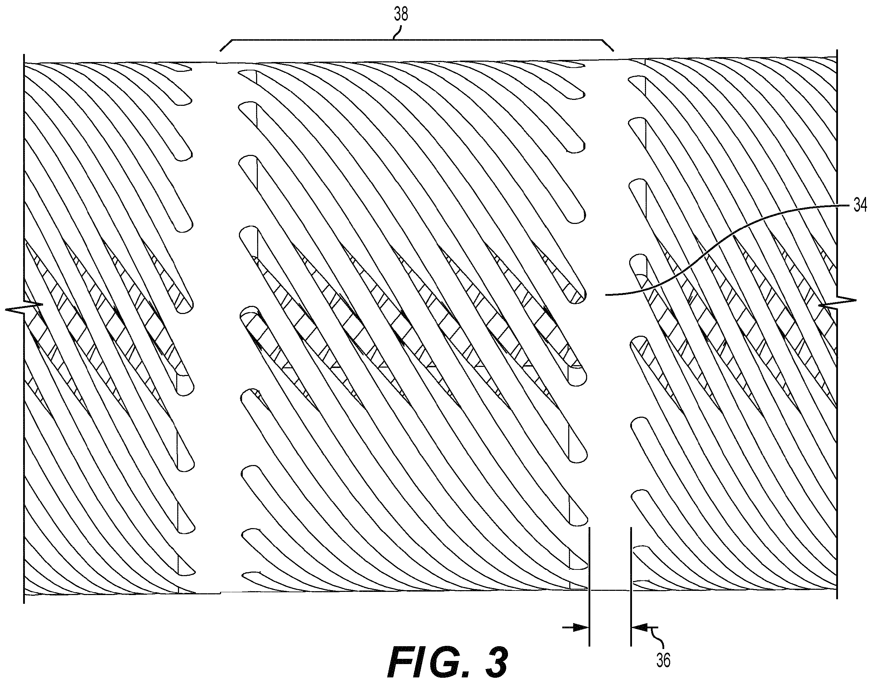

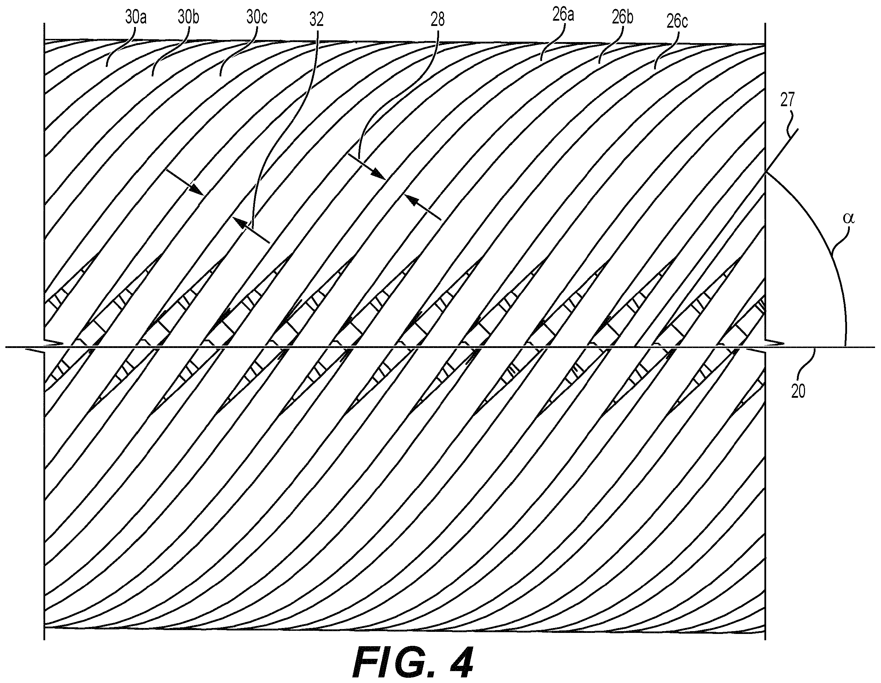

Now referring to the figures, FIGS. 1-6 illustrate examples of the device and component embodiments of an intravascular medical device 10, such as a catheter, constructed in accordance with the principles and advantages disclosed herein. The device 10 is a minimally-invasive device that can be introduced and operated percutaneously and intravascularly in conjunction with one or more other devices as disclosed herein, such as those used in interventional radiology, cardiology, and neuroradiologically to assess and/or treat occlusions, obstructions or other vascular defects, malformations and or conditions. Examples of the medical device 10 include but are not limited to catheters, micro catheters, balloon catheters, guide catheters, guide catheter extensions, dual lumen catheters, dissection and or subintimal re-entry catheters, aspiration catheters, device delivery catheters for vascular and structural heart valves, stent-grafts, stents, or vascular filters or the like. The medical device may be configured for passage in and through neurovascular, coronary, peripheral, and/or arterial pathways, and may also be configured for non-vascular endoscopic and/or laparoscopic applications.

The device 10 generally includes an elongated catheter body 12 with sufficient length, flexibility, and torqueability characteristics to be introduced and operated from an exterior of the patient, traverse the vasculature, and be positioned proximate the region being assessed or treated. The catheter body 12 generally includes a proximal segment 14 that may connect to and/or terminate at a hub or other component exterior to a patient, and a distal segment 16 (which may be fabricated from one continuous length of definable segments). The catheter body 12 further includes or defines a lumen 18 extending therethrough and exiting at the distal segment 16, where the lumen 18 has a diameter sufficient to pass a guidewire or other interventional devices such as coils or embolic materials therethrough and/or to introduce one or more other medical instruments or devices through the catheter body 12. The catheter body 12 may generally define a longitudinal axis 20 along a length thereof.

The catheter body 12 may be constructed at least in part from a polymer or metallic tube, hypotube, or other tubular or cylindrical construct having one or more segments constructed from metal, nitinol, stainless steel, polymers, polymer compositions and/or other materials and composites thereof. The catheter body 12 may constitute a unitary, single-piece construction, or may be modular and include multiple components of similar or varied composition of materials assembled, attached, or otherwise coupled together to form the body 12. Additional features of the device 10 and the catheter body 12 are provided in U.S. Patent Application Serial No. 15/726,024 (U.S. Pat. Pub. No. 2018/0093070), entitled 'MODULAR VASCULAR CATHETER,' and U.S. Patent Application Serial No. 16/255,141 (U.S. Pat. Pub. No. 2019/0160259), entitled 'VARIABLE FLEXIBILITY CATHETER SUPPORT FRAME.

The catheter body 12 may include one or more zones 22a, 22b, 22c...22z (collectively referred to as '22') along a length thereof that provide varying characteristics related to steerability, torqueability, variable bending flexibility, pushability, collapse or kink resistance, catheter shaft fatigue or resistance to plastic deformability, or the like. Each zone 22 may, for example, have varying multi-filar cut patterns, length, spacing between cut pattern segments, wall construction, material composition, or other design features different than the other zones 22 to provide a desired functionality of the device 10. The catheter body 12 may also be referred to, at least in part, as a catheter tube 12.

Each zone 22 may include one or more cut pattern segments 24a, 24b, 24c...24z (collectively referred to as '24') extending along a length thereof. The cut pattern segments 24 within a particular zone 22 may be substantially similar to other cut patterns or cut pattern segments 24 within that zone or may vary to provide a range of functional, progressive characteristics in distal or proximal directions within a single zone 22.

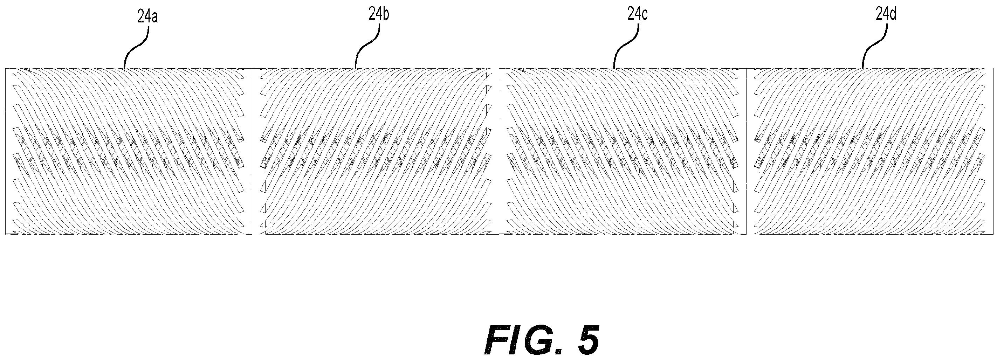

Each cut pattern segment 24 may include a cut pattern therein having a plurality of cuts or material otherwise removed or absent from the catheter body to provide a desired functionality of the device 10. For example, a cut pattern segment 24 may include or define a plurality of circumferentially spaced cuts 26a, 26b, 26c...26z (collectively referred to as '26'). The cuts 26 within a cut pattern segment 24 may be substantially similar to one another, or may vary within a single cut pattern segment 24. Each cut 26 may define a pitch angle α with respect to a pitch axis 27 and the longitudinal axis 20 of the catheter body 12, and may define a cut width 28 as measured perpendicular to the pitch angle axis. The direction of the pitch of the cuts 26 may vary amongst cut pattern segments 24 (i.e., counterclockwise vs. clockwise rotation of the pitch, as shown in FIG. 5), and the quantity of the cuts 26 within the circumference of a particular cut pattern segment 24 may also vary to provide the desired characteristics in a zone 22 and the spiral length of cut relative to the longitudinal axis or segment length.

Each cut pattern segment 24 may include a plurality of uncut portions or filars 30a, 30b, 30c ...30z (collectively referred to as '30') between the cuts 26 to provide a desired functionality of the device 10. The filars 30 within a cut pattern segment 24 may be substantially similar to one another, or may vary within a single cut pattern segment 24. Each filar 30 may have the same pitch angle α as the cuts 26 with respect to the longitudinal axis 20 of the catheter body 12, and may define a filar width 32 as measured perpendicular to the pitch angle axis. The direction of the pitch of the filars 30 may vary amongst cut pattern segments 24 (i.e., counterclockwise vs. clockwise rotation of the pitch), and the quantity of the filars 30 within the circumference of a particular cut pattern segment 24 may also vary to provide the desired characteristics in a zone 22. The disclosed multi-filar feature provides improved axial pull strength compared to prior art mono-filar or filament designs, among other benefits and improved function disclosed herein.

The catheter body 12 may include or define an uncut segment 34 positioned longitudinally between successive or adjacent pluralities of cuts 26. The uncut segment 34 may define a length 36 as measured along the longitudinal axis 20 to form an uncut region or ring in between successive or adjacent pluralities of cuts 26. The uncut segment(s) 34 may be composed of an interruption from one cut pattern to the next. For example, these interrupted cut segments may occur repeatedly along the length of the body 12 at substantially the same circumferential location about the center axis and restart the inline cut pattern into the next adjacent cut segment.

Each cut pattern segment 24 may define a length 38 as measured along the longitudinal axis 20, where the length 38 includes the length of the plurality of cuts 26 therein and half of each bordering uncut segment length 36 on either side of the cuts 26.

In one example, the catheter body 12 may include a first zone 22a at or near the distal portion of the medical device 10. The first zone 22a may include at least one cut pattern segment 24a having a plurality of circumferentially-spaced cuts 26 having a cut width between 0.015 mm and 0.03 mm, and may preferably be approximately 0.023 mm. The first zone 22a may define a plurality of circumferentially-spaced filars 30 having a width between 0.025 mm and 0.035 mm, and may preferably be approximately 0.030 mm.

The plurality of cuts 26 and the plurality of filars 30 may each have a pitch angle α between 60 degrees and 70 degrees, and may preferably be approximately 65 degrees. The cut pattern segment 24a may include between 16 and 24 cuts, and between 16 and 24 filars. The cut pattern segment 24a may preferably include 20 cuts, and 20 filars.

The first zone 22a may include a plurality of cut pattern segments having the dimensions specified above. The number of cut pattern segments in the first zone 22a may be between 1 and 40. Each cut pattern segment of the first zone 22a has a segment length between 1 mm and 1.3 mm, and may preferably be approximately 1.15 mm. The first zone 22a may include a plurality of uncut segments 34, with each uncut segment 34 defining an uncut segment length between 0.05 mm and 0.1 mm, and may preferably be approximately 0.075 mm.

This first zone 22a may have an average flexibility between approximately 0 gF/mm and approximately 0.75 gF/mm.

Continuing in this example of an exemplary medical device, the catheter body 12 may include a second zone 22b located proximal of the first zone 22a. The second zone 22b may include at least one cut pattern segment 24b having a plurality of circumferentially-spaced cuts 26 having a cut width between 0.015 mm and 0.03 mm, and may preferably be approximately 0.022 mm. The second zone 22b may define a plurality of circumferentially-spaced filars 30 having a width between 0.055 mm and 0.065 mm, and may preferably be approximately 0.06 mm.

The plurality of cuts 26 and the plurality of filars 30 may each have a pitch angle α between 45 degrees and 55 degrees, and may preferably be approximately 50 degrees. The cut pattern segment 24b may include between 16 and 24 cuts, and between 16 and 24 filars. The cut pattern segment 24b may preferably include 20 cuts, and 20 filars.

The second zone 22b may include a plurality of cut pattern segments having the dimensions specified above. The number of cut pattern segments in the second zone 22b may be between 1 and 40. Each cut pattern segment of the second zone 22b has a segment length between 2 mm and 2.3 mm, and may preferably be approximately 2.15 mm. The second zone 22b may include a plurality of uncut segments 34, with each uncut segment 34 defining an uncut segment length between 0.1 mm and 0.2 mm, and may preferably be approximately 0.15 mm.

This second zone 22b may have an average flexibility between approximately 0.75 gF/mm and approximately 1.25 gF/mm.

Continuing in this example, the catheter body 12 may include a third zone 22c located proximal of the second zone 22b. The third zone 22c may include at least one cut pattern segment 24c having a plurality of circumferentially-spaced cuts 26 having a cut width between 0.02 mm and 0.03 mm, and may preferably be approximately 0.023 mm. The third zone 22c may define a plurality of circumferentially-spaced filars 30 having a width between 0.08 mm and 0.09 mm, and may preferably be approximately 0.085 mm.

The plurality of cuts 26 and the plurality of filars 30 may each have a pitch angle α between 25 degrees and 40 degrees, and may preferably be approximately 32 degrees. The cut pattern segment 24c may include between 16 and 24 cuts, and between 16 and 24 filars. The cut pattern segment 24c may preferably include 20 cuts, and 20 filars.

The third zone 22c may include a plurality of cut pattern segments having the dimensions specified above. The number of cut pattern segments in the third zone 22c may be between 1 and 40. Each cut pattern segment of the third zone 22c has a segment length between 3 mm and 5 mm, and may preferably be approximately 4 mm. The third zone 22c may include a plurality of uncut segments 34, with each uncut segment 34 defining an uncut segment length between 0.10 mm and 0.25 mm, and may preferably be approximately 0.17 mm.

This third zone 22c may have an average flexibility between approximately 1.25 gF/mm and approximately 1.75 gF/mm.

In one example, the catheter body 12 may have an inner diameter between 0.254 mm and 1.27 mm, and outer diameter between 0.0508 mm and 0.254 mm, and/or a wall thickness between 0.051 mm and 0.102 mm. Variations of the dimensions and patterns described herein may be incorporated into devices of all applicable inner and outer diameters, including those ranging from 3 French to 34 French.

In one example, the catheter body 12 may include a first zone 22a at or near the distal portion of the medical device 10. The first zone 22a may include at least one cut pattern segment 24a having a plurality of circumferentially-spaced cuts 26 having a cut width between 0.015 mm and 0.25 mm, and may preferably be approximately 0.02 mm. The first zone 22a may define a plurality of circumferentially-spaced filars 30 having a width between 0.03 mm and 0.04 mm, and may preferably be approximately 0.034 mm.

The plurality of cuts 26 and the plurality of filars 30 may each have a pitch angle α between 60 degrees and 70 degrees, and may preferably be approximately 64 degrees. The cut pattern segment 24a may include between 16 and 24 cuts, and between 16 and 24 filars. The cut pattern segment 24a may preferably include 20 cuts, and 20 filars.

The first zone 22a may include a plurality of cut pattern segments having the dimensions specified above. The number of cut pattern segments in the first zone 22a may be between 1 and 40. Each cut pattern segment of the first zone 22a has a segment length between 1 mm and 1.3 mm, and may preferably be approximately 1.15 mm. The first zone 22a may include a plurality of uncut segments 34, with each uncut segment 34 defining an uncut segment length between 0.05 mm and 0.1 mm, and may preferably be approximately 0.08 mm.

This first zone 22a may have an average flexibility between approximately 0 gF/mm and approximately 0.75 gF/mm.

Continuing in this example of an exemplary medical device, the catheter body 12 may include a second zone 22b located proximal of the first zone 22a. The second zone 22b may include at least one cut pattern segment 24b having a plurality of circumferentially-spaced cuts 26 having a cut width between 0.015 mm and 0.025 mm, and may preferably be approximately 0.02 mm. The second zone 22b may define a plurality of circumferentially-spaced filars 30 having a width between 0.055 mm and 0.065 mm, and may preferably be approximately 0.06 mm.

The plurality of cuts 26 and the plurality of filars 30 may each have a pitch angle α between 45 degrees and 55 degrees, and may preferably be approximately 48 degrees. The cut pattern segment 24b may include between 16 and 24 cuts, and between 16 and 24 filars. The cut pattern segment 24b may preferably include 20 cuts, and 20 filars.

The second zone 22b may include a plurality of cut pattern segments having the dimensions specified above. The number of cut pattern segments in the second zone 22b may be between 1 and 40. Each cut pattern segment of the second zone 22b has a segment length between 2 mm and 2.3 mm, and may preferably be approximately 2.15 mm. The second zone 22b may include a plurality of uncut segments 34, with each uncut segment 34 defining an uncut segment length between 0.1 mm and 0.2 mm, and may preferably be approximately 0.15 mm.

This second zone 22b may have an average flexibility between approximately 0.75 gF/mm and approximately 1.25 gF/mm.

Continuing in this example, the catheter body 12 may include a third zone 22c located proximal of the second zone 22b. The third zone 22c may include at least one cut pattern segment 24c having a plurality of circumferentially-spaced cuts 26 having a cut width between 0.02 mm and 0.03 mm, and may preferably be approximately 0.023 mm. The third zone 22c may define a plurality of circumferentially-spaced filars 30 having a width between 0.07 mm and 0.09 mm, and may preferably be approximately 0.08 mm.

The plurality of cuts 26 and the plurality of filars 30 may each have a pitch angle α between 25 degrees and 40 degrees, and may preferably be approximately 32 degrees. The cut pattern segment 24c may include between 16 and 24 cuts, and between 16 and 24 filars. The cut pattern segment 24c may preferably include 20 cuts, and 20 filars.

The third zone 22c may include a plurality of cut pattern segments having the dimensions specified above. The number of cut pattern segments in the third zone 22c may be between 1 and 40. Each cut pattern segment of the third zone 22c has a segment length between 3 mm and 5 mm, and may preferably be approximately 4 mm. The third zone 22c may include a plurality of uncut segments 34, with each uncut segment 34 defining an uncut segment length between 0.10 mm and 0.25 mm, and may preferably be approximately 0.18 mm.

This third zone 22c may have an average flexibility between approximately 1.25 gF/mm and approximately 1.75 gF/mm.

In one example, the catheter body 12 may have an inner diameter between 0.254 mm and 1.27 mm, and outer diameter between 0.0508 mm and 0.254 mm, and/or a wall thickness between 0.051 mm and 0.102 mm.

In another example, the catheter body 12 may have an inner diameter between 0.45 mm and 0.52 mm, and outer diameter between 0.6 mm and 0.7 mm, and/or a wall thickness between 0.07 mm and 0.1 mm. Variations of the dimensions and patterns described herein may be incorporated into devices of all applicable inner and outer diameters, including those ranging from 3 French to 34 French.

In this example, the catheter body 12 may include a first zone 22a at or near the distal portion of the medical device 10. The first zone 22a may include at least one cut pattern segment 24a having a plurality of circumferentially-spaced cuts 26 having a cut width between 0.018 mm and 0.026 mm, and may preferably be approximately 0.022 mm. The first zone 22a may define a plurality of circumferentially-spaced filars 30 having a width between 0.025 mm and 0.037 mm, and may preferably be approximately 0.031 mm.

The plurality of cuts 26 and the plurality of filars 30 may each have a pitch angle α between 58 degrees and 68 degrees, and may preferably be approximately 63 degrees. The cut pattern segment 24a may include between 12 and 22 cuts, and between 12 and 22 filars. The cut pattern segment 24a may preferably include 17 cuts, and 17 filars.

The first zone 22a may include a plurality of cut pattern segments having the dimensions specified above. The number of cut pattern segments in the first zone 22a may be between 1 and 40. Each cut pattern segment of the first zone 22a has a segment length between 1.0 mm and 1.3 mm, and may preferably be approximately 1.15 mm. The first zone 22a may include a plurality of uncut segments 34, with each uncut segment 34 defining an uncut segment length between 0.06 mm and 0.1 mm, and may preferably be approximately 0.08 mm.

This first zone 22a may have an average flexibility between approximately 0.1 gF/mm and approximately 0.16 gF/mm.

Continuing in this example of an exemplary medical device, the catheter body 12 may include a second zone 22b located proximal of the first zone 22a. The second zone 22b may include at least one cut pattern segment 24b having a plurality of circumferentially-spaced cuts 26 having a cut width between 0.018 mm and 0.026 mm, and may preferably be approximately 0.022 mm. The second zone 22b may define a plurality of circumferentially-spaced filars 30 having a width between 0.05 mm and 0.07 mm, and may preferably be approximately 0.06 mm.

The plurality of cuts 26 and the plurality of filars 30 may each have a pitch angle α between 44 degrees and 54 degrees, and may preferably be approximately 49 degrees. The cut pattern segment 24b may include between 12 and 22 cuts, and between 12 and 22 filars. The cut pattern segment 24b may preferably include 17 cuts, and 17 filars.

The second zone 22b may include a plurality of cut pattern segments having the dimensions specified above. The number of cut pattern segments in the second zone 22b may be between 20 and 80. Each cut pattern segment of the second zone 22b has a segment length between 2.0 mm and 2.3 mm, and may preferably be approximately 2.14 mm. The second zone 22b may include a plurality of uncut segments 34, with each uncut segment 34 defining an uncut segment length between 0.08 mm and .18 mm, and may preferably be approximately 0.13 mm.

This second zone 22b may have an average flexibility between approximately 0.16 gF/mm and approximately 0.55 gF/mm.

Continuing in this example, the catheter body 12 may include a third zone 22c located proximal of the second zone 22b. The third zone 22c may include at least one cut pattern segment 24c having a plurality of circumferentially-spaced cuts 26 having a cut width between between 0.018 mm and 0.026 mm, and may preferably be approximately 0.023 mm. The third zone 22c may define a plurality of circumferentially-spaced filars 30 having a width between 0.05 mm and 0.12 mm, and may preferably be approximately 0.085 mm.

The plurality of cuts 26 and the plurality of filars 30 may each have a pitch angle α between 27 degrees and 37 degrees, and may preferably be approximately 32 degrees. The cut pattern segment 24c may include between 12 and 22 cuts, and between 12 and 22 filars. The cut pattern segment 24c may preferably include 17 cuts, and 17 filars.

The third zone 22c may include a plurality of cut pattern segments having the dimensions specified above. The number of cut pattern segments in the third zone 22c may be between 50 and 150. Each cut pattern segment of the third zone 22c has a segment length between 3.5 mm and 4.5 mm, and may preferably be approximately 3.95 mm. The third zone 22c may include a plurality of uncut segments 34, with each uncut segment 34 defining an uncut segment length between 0.12 mm and 0.22 mm, and may preferably be approximately 0.17 mm.

This third zone 22c may have an average flexibility between approximately 0.56 gF/mm and approximately 0.88 gF/mm.

In another example, the catheter body 12 may have an inner diameter between 0.4 mm and 0.55 mm, and outer diameter between 0.65 mm and 0.78 mm, and/or a wall thickness between 0.05 mm and 0.11 mm. Variations of the dimensions and patterns described herein may be incorporated into devices of all applicable inner and outer diameters, including those ranging from 3 French to 34 French.

In this example, the catheter body 12 may include a first zone 22a at or near the distal portion of the medical device 10. The first zone 22a may include at least one cut pattern segment 24a having a plurality of circumferentially-spaced cuts 26 having a cut width between 0.018 mm and 0.026 mm, and may preferably be approximately 0.022 mm. The first zone 22a may define a plurality of circumferentially-spaced filars 30 having a width between 0.04 mm and 0.07 mm, and may preferably be approximately 0.055 mm.

The plurality of cuts 26 and the plurality of filars 30 may each have a pitch angle α between 70 degrees and 80 degrees, and may preferably be approximately 75 degrees. The cut pattern segment 24a may include between 5 and 10 cuts, and between 5 and 10 filars. The cut pattern segment 24a may preferably include 7 cuts, and 7 filars.

The first zone 22a may include a plurality of cut pattern segments having the dimensions specified above. The number of cut pattern segments in the first zone 22a may be between 1 and 40. Each cut pattern segment of the first zone 22a has a segment length between 1.0 mm and 2.0 mm, and may preferably be approximately 1.5 mm. The first zone 22a may include a plurality of uncut segments 34, with each uncut segment 34 defining an uncut segment length between 0.05 mm and 0.15 mm, and may preferably be approximately 0.11 mm.

This first zone 22a may have an average flexibility between approximately 0.1 gF/mm and approximately 0.16 gF/mm.

Continuing in this example of an exemplary medical device, the catheter body 12 may include a second zone 22b located proximal of the first zone 22a. The second zone 22b may include at least one cut pattern segment 24b having a plurality of circumferentially-spaced cuts 26 having a cut width between 0.018 mm and 0.026 mm, and may preferably be approximately 0.022 mm. The second zone 22b may define a plurality of circumferentially-spaced filars 30 having a width between 0.045 mm and 0.075 mm, and may preferably be approximately 0.06 mm.

The plurality of cuts 26 and the plurality of filars 30 may each have a pitch angle α between 44 degrees and 54 degrees, and may preferably be approximately 49 degrees. The cut pattern segment 24b may include between 12 and 22 cuts, and between 12 and 22 filars. The cut pattern segment 24b may preferably include 17 cuts, and 17 filars.

The second zone 22b may include a plurality of cut pattern segments having the dimensions specified above. The number of cut pattern segments in the second zone 22b may be between 25 and 75. Each cut pattern segment of the second zone 22b has a segment length between 2.0 mm and 2.3 mm, and may preferably be approximately 2.14 mm. The second zone 22b may include a plurality of uncut segments 34, with each uncut segment 34 defining an uncut segment length between 0.10 mm and 0.17 mm, and may preferably be approximately 0.13 mm.

This second zone 22b may have an average flexibility between approximately 0.16 gF/mm and approximately 0.56 gF/mm.

Continuing in this example, the catheter body 12 may include a third zone 22c located proximal of the second zone 22b. The third zone 22c may include at least one cut pattern segment 24c having a plurality of circumferentially-spaced cuts 26 having a cut width between 0.018 mm and 0.026 mm, and may preferably be approximately 0.023 mm. The third zone 22c may define a plurality of circumferentially-spaced filars 30 having a width between 0.07 mm and 0.10 mm, and may preferably be approximately 0.085 mm.

The plurality of cuts 26 and the plurality of filars 30 may each have a pitch angle α between 27 degrees and 37 degrees, and may preferably be approximately 32 degrees. The cut pattern segment 24c may include between 12 and 22 cuts, and between 12 and 22 filars. The cut pattern segment 24c may preferably include 17 cuts, and 17 filars.

The third zone 22c may include a plurality of cut pattern segments having the dimensions specified above. The number of cut pattern segments in the third zone 22c may be between 50 and 150. Each cut pattern segment of the third zone 22c has a segment length between 3.0 mm and 5.0 mm, and may preferably be approximately 3.95 mm. The third zone 22c may include a plurality of uncut segments 34, with each uncut segment 34 defining an uncut segment length between 0.1 mm and 0.3 mm, and may preferably be approximately 0.17 mm.

This third zone 22c may have an average flexibility between approximately 0.55 gF/mm and approximately 0.88 gF/mm.

In another example, the catheter body 12 may have an inner diameter between 0.43 mm and 0.63 mm, and outer diameter between 0.66 mm and 0.86 mm, and/or a wall thickness between 0.06 mm and 0.17 mm. Variations of the dimensions and patterns described herein may be incorporated into devices of all applicable inner and outer diameters, including those ranging from 3 French to 34 French.

In this example, the catheter body 12 may include a first zone 22a at or near the distal portion of the medical device 10. The first zone 22a may include at least one cut pattern segment 24a having a plurality of circumferentially-spaced cuts 26 having a cut width between 0.015 mm and 0.024 mm, and may preferably be approximately 0.019 mm. The first zone 22a may define a plurality of circumferentially-spaced filars 30 having a width between 0.025 mm and 0.045 mm, and may preferably be approximately 0.34 mm.

The plurality of cuts 26 and the plurality of filars 30 may each have a pitch angle α between 59 degrees and 69 degrees, and may preferably be approximately 64 degrees. The cut pattern segment 24a may include between 15 and 25 cuts, and between 15 and 25 filars. The cut pattern segment 24a may preferably include 20 cuts, and 20 filars.

The first zone 22a may include a plurality of cut pattern segments having the dimensions specified above. The number of cut pattern segments in the first zone 22a may be between 1 and 40. Each cut pattern segment of the first zone 22a has a segment length between 1.0 mm and 1.3 mm, and may preferably be approximately 1.14 mm. The first zone 22a may include a plurality of uncut segments 34, with each uncut segment 34 defining an uncut segment length between 0.07 mm and 0.09 mm, and may preferably be approximately 0.079 mm.

This first zone 22a may have an average flexibility between approximately 0.1 gF/mm and approximately 0.24 gF/mm.

Continuing in this example of an exemplary medical device, the catheter body 12 may include a second zone 22b located proximal of the first zone 22a. The second zone 22b may include at least one cut pattern segment 24b having a plurality of circumferentially-spaced cuts 26 having a cut width between 0.015 mm and 0.024 mm, and may preferably be approximately 0.019 mm. The second zone 22b may define a plurality of circumferentially-spaced filars 30 having a width between 0.05 mm and 0.07 mm, and may preferably be approximately 0.059 mm.

The plurality of cuts 26 and the plurality of filars 30 may each have a pitch angle α between 43 degrees and 53 degrees, and may preferably be approximately 48 degrees. The cut pattern segment 24b may include between 16 and 24 cuts, and between 16 and 24 filars. The cut pattern segment 24b may preferably include 20 cuts, and 20 filars.

The second zone 22b may include a plurality of cut pattern segments having the dimensions specified above. The number of cut pattern segments in the second zone 22b may be between 25 and 75. Each cut pattern segment of the second zone 22b has a segment length between 2.0 mm and 2.3 mm, and may preferably be approximately 2.14 mm. The second zone 22b may include a plurality of uncut segments 34, with each uncut segment 34 defining an uncut segment length between 0.10 mm and 0.20 mm, and may preferably be approximately 0.14 mm.

This second zone 22b may have an average flexibility between approximately 0.24 gF/mm and approximately 0.80 gF/mm.

Continuing in this example, the catheter body 12 may include a third zone 22c located proximal of the second zone 22b. The third zone 22c may include at least one cut pattern segment 24c having a plurality of circumferentially-spaced cuts 26 having a cut width between 0.018 mm and 0.028 mm, and may preferably be approximately 0.023 mm. The third zone 22c may define a plurality of circumferentially-spaced filars 30 having a width between 0.07 mm and 0.09 mm, and may preferably be approximately 0.79 mm.

The plurality of cuts 26 and the plurality of filars 30 may each have a pitch angle α between 28 degrees and 38 degrees, and may preferably be approximately 33 degrees. The cut pattern segment 24c may include between 16 and 24 cuts, and between 16 and 24 filars. The cut pattern segment 24c may preferably include 20 cuts, and 20 filars.

The third zone 22c may include a plurality of cut pattern segments having the dimensions specified above. The number of cut pattern segments in the third zone 22c may be between 75 and 150. Each cut pattern segment of the third zone 22c has a segment length between 3.5 mm and 4.5 mm, and may preferably be approximately 4.12 mm. The third zone 22c may include a plurality of uncut segments 34, with each uncut segment 34 defining an uncut segment length between 0.1 mm and 0.3 mm, and may preferably be approximately 0.18 mm.

This third zone 22c may have an average flexibility between approximately 0.80 gF/mm and approximately 2.4 gF/mm.

In another example, the catheter body 12 may have an inner diameter between 0.45 mm and 0.65 mm, and outer diameter between 0.7 mm and 0.8 mm, and/or a wall thickness between 0.08 mm and 0.13 mm. Variations of the dimensions and patterns described herein may be incorporated into devices of all applicable inner and outer diameters, including those ranging from 3 French to 34 French.

In this example, the catheter body 12 may include a first zone 22a at or near the distal portion of the medical device 10. The first zone 22a may include at least one cut pattern segment 24a having a plurality of circumferentially-spaced cuts 26 having a cut width between 0.025 mm and 0.035 mm, and may preferably be approximately 0.03 mm. The first zone 22a may define a plurality of circumferentially-spaced filars 30 having a width between 0.022 mm and 0.032 mm, and may preferably be approximately 0.027 mm.

The plurality of cuts 26 and the plurality of filars 30 may each have a pitch angle α between 59 degrees and 69 degrees, and may preferably be approximately 64 degrees. The cut pattern segment 24a may include between 16 and 24 cuts, and between 16 and 24 filars. The cut pattern segment 24a may preferably include 20 cuts, and 20 filars.

The first zone 22a may include a plurality of cut pattern segments having the dimensions specified above. The number of cut pattern segments in the first zone 22a may be between 1 and 40. Each cut pattern segment of the first zone 22a has a segment length between 1.15 mm and 1.35 mm, and may preferably be approximately 1.25 mm. The first zone 22a may include a plurality of uncut segments 34, with each uncut segment 34 defining an uncut segment length between 0.05 mm and 0.15 mm, and may preferably be approximately 0.1 mm.

This first zone 22a may have an average flexibility between approximately 0.01 gF/mm and approximately 0.08 gF/mm.

Continuing in this example of an exemplary medical device, the catheter body 12 may include a second zone 22b located proximal of the first zone 22a. The second zone 22b may include at least one cut pattern segment 24b having a plurality of circumferentially-spaced cuts 26 having a cut width between 0.027 mm and 0.037 mm, and may preferably be approximately 0.032 mm. The second zone 22b may define a plurality of circumferentially-spaced filars 30 having a width between 0.038 mm and 0.048 mm, and may preferably be approximately 0.043 mm.

The plurality of cuts 26 and the plurality of filars 30 may each have a pitch angle α between 45 degrees and 55 degrees, and may preferably be approximately 50 degrees. The cut pattern segment 24b may include between 16 and 24 cuts, and between 16 and 24 filars. The cut pattern segment 24b may preferably include 20 cuts, and 20 filars.

The second zone 22b may include a plurality of cut pattern segments having the dimensions specified above. The number of cut pattern segments in the second zone 22b may be between 1 and 40. Each cut pattern segment of the second zone 22b has a segment length between 1.25 mm and 1.75 mm, and may preferably be approximately 1.52 mm. The second zone 22b may include a plurality of uncut segments 34, with each uncut segment 34 defining an uncut segment length between 0.05 mm and 0.15 mm, and may preferably be approximately 0.1 mm.

This second zone 22b may have an average flexibility between approximately 0.08 gF/mm and approximately 0.24 gF/mm.

Continuing in this example, the catheter body 12 may include a third zone 22c located proximal of the second zone 22b. The third zone 22c may include at least one cut pattern segment 24c having a plurality of circumferentially-spaced cuts 26 having a cut width between 0.02 mm and 0.04 mm, and may preferably be approximately 0.031 mm. The third zone 22c may define a plurality of circumferentially-spaced filars 30 having a width between 0.055 mm and 0.075 mm, and may preferably be approximately 0.064 mm.

The plurality of cuts 26 and the plurality of filars 30 may each have a pitch angle α between 40 degrees and 50 degrees, and may preferably be approximately 45 degrees. The cut pattern segment 24c may include between 14 and 22 cuts, and between 14 and 22 filars. The cut pattern segment 24c may preferably include 18 cuts, and 18 filars.

The third zone 22c may include a plurality of cut pattern segments having the dimensions specified above. The number of cut pattern segments in the third zone 22c may be between 1 and 40. Each cut pattern segment of the third zone 22c has a segment length between 1.4 mm and 1.6 mm, and may preferably be approximately 1.52 mm. The third zone 22c may include a plurality of uncut segments 34, with each uncut segment 34 defining an uncut segment length between 0.05 mm and 0.15 mm, and may preferably be approximately 0.1 mm.

This third zone 22c may have an average flexibility between approximately 0.24 gF/mm and approximately 1.04 gF/mm.

Continuing in this example of an exemplary medical device, the catheter body 12 may include a fourth zone 22d located proximal of the first zone 22a. The fourth zone 22d may include at least one cut pattern segment 24d having a plurality of circumferentially-spaced cuts 26 having a cut width between 0.02 mm and 0.04 mm, and may preferably be approximately 0.28 mm. The fourth zone 22b may define a plurality of circumferentially-spaced filars 30 having a width between 0.06 mm and 0.08 mm, and may preferably be approximately 0.072 mm.

The plurality of cuts 26 and the plurality of filars 30 may each have a pitch angle α between 35 degrees and 45 degrees, and may preferably be approximately 40 degrees. The cut pattern segment 24d may include between 16 and 24 cuts, and between 16 and 24 filars. The cut pattern segment 24d may preferably include 18 cuts, and 18 filars.

The fourth zone 22d may include a plurality of cut pattern segments having the dimensions specified above. The number of cut pattern segments in the fourth zone 22d may be between 1 and 40. Each cut pattern segment of the fourth zone 22d has a segment length between 1.25 mm and 1.75 mm, and may preferably be approximately 1.52 mm. The fourth zone 22d may include a plurality of uncut segments 34, with each uncut segment 34 defining an uncut segment length between 0.05 mm and 0.15 mm, and may preferably be approximately 0.1 mm.

This fourth zone 22b may have an average flexibility between approximately 1.04 gF/mm and approximately 1.68 gF/mm.

Continuing in this example, the catheter body 12 may include a fifth zone 22e located proximal of the fourth zone 22e. The fifth zone 22e may include at least one cut pattern segment 24e having a plurality of circumferentially-spaced cuts 26 having a cut width between 0.02 mm and 0.04 mm, and may preferably be approximately 0.03 mm. The fifth zone 22e may define a plurality of circumferentially-spaced filars 30 having a width between 0.07 mm and 0.09 mm, and may preferably be approximately 0.08 mm.

The plurality of cuts 26 and the plurality of filars 30 may each have a pitch angle α between 25 degrees and 35 degrees, and may preferably be approximately 30 degrees. The cut pattern segment 24e may include between 16 and 24 cuts, and between 16 and 24 filars. The cut pattern segment 24e may preferably include 18 cuts, and 18 filars.

The fifth zone 22e may include a plurality of cut pattern segments having the dimensions specified above. The number of cut pattern segments in the fifth zone 22e may be between 1 and 40. Each cut pattern segment of the fifth zone 22e has a segment length between 1.25 mm and 1.75 mm, and may preferably be approximately 1.53 mm. The fifth zone 22e may include a plurality of uncut segments 34, with each uncut segment 34 defining an uncut segment length between 0.05 mm and 0.15 mm, and may preferably be approximately 0.1 mm.

This fifth zone 22e may have an average flexibility between approximately 1.68 gF/mm and approximately 3.68 gF/mm.

Continuing in this example of an exemplary medical device, the catheter body 12 may include a sixth zone 22f located proximal of the fifth zone 22e. The sixth zone 22f may include at least one cut pattern segment 24f having a plurality of circumferentially-spaced cuts 26 having a cut width between 0.02 mm and 0.04 mm, and may preferably be approximately 0.028 mm. The sixth zone 22f may define a plurality of circumferentially-spaced filars 30 having a width between 0.07 mm and 0.1 mm, and may preferably be approximately 0.089 mm.

The plurality of cuts 26 and the plurality of filars 30 may each have a pitch angle α between 22 degrees and 32 degrees, and may preferably be approximately 27 degrees. The cut pattern segment 24f may include between 16 and 24 cuts, and between 16 and 24 filars. The cut pattern segment 24f may preferably include 18 cuts, and 18 filars.

The sixth zone 22f may include a plurality of cut pattern segments having the dimensions specified above. The number of cut pattern segments in the sixth zone 22f may be between 1 and 40. Each cut pattern segment of the sixth zone 22f has a segment length between 1.25 mm and 1.75 mm, and may preferably be approximately 1.52 mm. The sixth zone 22f may include a plurality of uncut segments 34, with each uncut segment 34 defining an uncut segment length between 0.05 mm and 0.15 mm, and may preferably be approximately 0.1 mm.

This sixth zone 22f may have an average flexibility between approximately 3.68 gF/mm and approximately 4.56 gF/mm.

Continuing in this example, the catheter body 12 may include a seventh zone 22g located proximal of the sixth zone 22f. The seventh zone 22g may include at least one cut pattern segment 24g having a plurality of circumferentially-spaced cuts 26 having a cut width between 0.02 mm and 0.04 mm, and may preferably be approximately 0.029 mm. The seventh zone 22g may define a plurality of circumferentially-spaced filars 30 having a width between 0.075 mm and 0.10 mm, and may preferably be approximately 0.091 mm.

The plurality of cuts 26 and the plurality of filars 30 may each have a pitch angle α between 15 degrees and 25 degrees, and may preferably be approximately 20 degrees. The cut pattern segment 24g may include between 16 and 24 cuts, and between 16 and 24 filars. The cut pattern segment 24g may preferably include 18 cuts, and 18 filars.

The seventh zone 22g may include a plurality of cut pattern segments having the dimensions specified above. The number of cut pattern segments in the seventh zone 22g may be between 1 and 40. Each cut pattern segment of the seventh zone 22g has a segment length between 1.25 mm and 1.75 mm, and may preferably be approximately 1.52 mm. The seventh zone 22g may include a plurality of uncut segments 34, with each uncut segment 34 defining an uncut segment length between 0.05 mm and 0.15 mm, and may preferably be approximately 0.1 mm.

This seventh zone 22g may have an average flexibility between approximately 4.56 gF/mm and approximately 6.08 gF/mm.

In another example, the catheter body 12 may have an inner diameter between 0.45 mm and 0.65 mm, and outer diameter between 0.7 mm and 0.8 mm, and/or a wall thickness between 0.08 mm and 0.13 mm. Variations of the dimensions and patterns described herein may be incorporated into devices of all applicable inner and outer diameters, including those ranging from 3 French to 34 French.

In this example, the catheter body 12 may include a first zone 22a at or near the distal portion of the medical device 10. The first zone 22a may include at least one cut pattern segment 24a having a plurality of circumferentially-spaced cuts 26 having a cut width between 0.01 mm and 0.03 mm, and may preferably be approximately 0.02 mm. The first zone 22a may define a plurality of circumferentially-spaced filars 30 having a width between 0.04 mm and 0.06 mm, and may preferably be approximately 0.049 mm.

The plurality of cuts 26 and the plurality of filars 30 may each have a pitch angle α between 55 degrees and 65 degrees, and may preferably be approximately 60 degrees. The cut pattern segment 24a may include between 16 and 24 cuts, and between 16 and 24 filars. The cut pattern segment 24a may preferably include 20 cuts, and 20 filars.

The first zone 22a may include a plurality of cut pattern segments having the dimensions specified above. The number of cut pattern segments in the first zone 22a may be between 1 and 40. Each cut pattern segment of the first zone 22a has a segment length between 1.0 mm and 1.5 mm, and may preferably be approximately 1.24 mm. The first zone 22a may include a plurality of uncut segments 34, with each uncut segment 34 defining an uncut segment length between 0.05 mm and 0.15 mm, and may preferably be approximately 0.1 mm.

This first zone 22a may have an average flexibility between approximately 0.008 gF/mm and approximately 0.16 gF/mm.

Continuing in this example of an exemplary medical device, the catheter body 12 may include a second zone 22b located proximal of the first zone 22a. The second zone 22b may include at least one cut pattern segment 24b having a plurality of circumferentially-spaced cuts 26 having a cut width between 0.01 mm and 0.03 mm, and may preferably be approximately 0.02 mm. The second zone 22b may define a plurality of circumferentially-spaced filars 30 having a width between 0.05 mm and 0.07 mm, and may preferably be approximately 0.057 mm.

The plurality of cuts 26 and the plurality of filars 30 may each have a pitch angle α between 45 degrees and 55 degrees, and may preferably be approximately 50 degrees. The cut pattern segment 24b may include between 16 and 24 cuts, and between 16 and 24 filars. The cut pattern segment 24b may preferably include 20 cuts, and 20 filars.

The second zone 22b may include a plurality of cut pattern segments having the dimensions specified above. The number of cut pattern segments in the second zone 22b may be between 1 and 40. Each cut pattern segment of the second zone 22b has a segment length between 1.25 mm and 1.75 mm, and may preferably be approximately 1.5 mm. The second zone 22b may include a plurality of uncut segments 34, with each uncut segment 34 defining an uncut segment length between 0.05 mm and 0.15 mm, and may preferably be approximately 0.1 mm.

This second zone 22b may have an average flexibility between approximately 0.16 gF/mm and approximately 0.48 gF/mm.

Continuing in this example, the catheter body 12 may include a third zone 22c located proximal of the second zone 22b. The third zone 22c may include at least one cut pattern segment 24c having a plurality of circumferentially-spaced cuts 26 having a cut width between 0.01 mm and 0.03 mm, and may preferably be approximately 0.02 mm. The third zone 22c may define a plurality of circumferentially-spaced filars 30 having a width between 0.06 mm and 0.09 mm, and may preferably be approximately 0.074 mm.

The plurality of cuts 26 and the plurality of filars 30 may each have a pitch angle α between 40 degrees and 50 degrees, and may preferably be approximately 45 degrees. The cut pattern segment 24c may include between 14 and 22 cuts, and between 14 and 22 filars. The cut pattern segment 24c may preferably include 18 cuts, and 18 filars.

The third zone 22c may include a plurality of cut pattern segments having the dimensions specified above. The number of cut pattern segments in the third zone 22c may be between 1 and 40. Each cut pattern segment of the third zone 22c has a segment length between 1.25 mm and 1.75 mm, and may preferably be approximately 1.5 mm. The third zone 22c may include a plurality of uncut segments 34, with each uncut segment 34 defining an uncut segment length between 0.05 mm and 0.15 mm, and may preferably be approximately 0.1 mm.

This third zone 22c may have an average flexibility between approximately 0.48 gF/mm and approximately 1.04 gF/mm.

Continuing in this example of an exemplary medical device, the catheter body 12 may include a fourth zone 22d located proximal of the first zone 22a. The fourth zone 22d may include at least one cut pattern segment 24d having a plurality of circumferentially-spaced cuts 26 having a cut width between 0.01 mm and 0.03 mm, and may preferably be approximately 0.2 mm. The fourth zone 22b may define a plurality of circumferentially-spaced filars 30 having a width between 0.07 mm and 0.09 mm, and may preferably be approximately 0.082 mm.

The plurality of cuts 26 and the plurality of filars 30 may each have a pitch angle α between 35 degrees and 45 degrees, and may preferably be approximately 40 degrees. The cut pattern segment 24d may include between 16 and 24 cuts, and between 16 and 24 filars. The cut pattern segment 24d may preferably include 18 cuts, and 18 filars.

The fourth zone 22d may include a plurality of cut pattern segments having the dimensions specified above. The number of cut pattern segments in the fourth zone 22d may be between 1 and 40. Each cut pattern segment of the fourth zone 22d has a segment length between 1.25 mm and 1.75 mm, and may preferably be approximately 1.5 mm. The fourth zone 22d may include a plurality of uncut segments 34, with each uncut segment 34 defining an uncut segment length between 0.05 mm and 0.15 mm, and may preferably be approximately 0.1 mm.

This fourth zone 22d may have an average flexibility between approximately 1.04 gF/mm and approximately 1.84 gF/mm.

Continuing in this example, the catheter body 12 may include a fifth zone 22e located proximal of the fourth zone 22e. The fifth zone 22e may include at least one cut pattern segment 24e having a plurality of circumferentially-spaced cuts 26 having a cut width between 0.01 mm and 0.03 mm, and may preferably be approximately 0.02 mm. The fifth zone 22e may define a plurality of circumferentially-spaced filars 30 having a width between 0.07 mm and 0.11 mm, and may preferably be approximately 0.09 mm.

The plurality of cuts 26 and the plurality of filars 30 may each have a pitch angle α between 28 degrees and 38 degrees, and may preferably be approximately 33 degrees. The cut pattern segment 24e may include between 12 and 24 cuts, and between 12 and 24 filars. The cut pattern segment 24e may preferably include 18 cuts, and 18 filars.

The fifth zone 22e may include a plurality of cut pattern segments having the dimensions specified above. The number of cut pattern segments in the fifth zone 22e may be between 1 and 40. Each cut pattern segment of the fifth zone 22e has a segment length between 1.25 mm and 1.75 mm, and may preferably be approximately 1.53 mm. The fifth zone 22e may include a plurality of uncut segments 34, with each uncut segment 34 defining an uncut segment length between 0.05 mm and 0.15 mm, and may preferably be approximately 0.1 mm.

This fifth zone 22e may have an average flexibility between approximately 1.84 gF/mm and approximately 3.6 gF/mm.

Continuing in this example of an exemplary medical device, the catheter body 12 may include a sixth zone 22f located proximal of the fifth zone 22e. The sixth zone 22f may include at least one cut pattern segment 24f having a plurality of circumferentially-spaced cuts 26 having a cut width between 0.01 mm and 0.03 mm, and may preferably be approximately 0.02 mm. The sixth zone 22f may define a plurality of circumferentially-spaced filars 30 having a width between 0.08 mm and 0.12 mm, and may preferably be approximately 0.098 mm.

The plurality of cuts 26 and the plurality of filars 30 may each have a pitch angle α between 22 degrees and 32 degrees, and may preferably be approximately 27 degrees. The cut pattern segment 24f may include between 12 and 24 cuts, and between 12 and 24 filars. The cut pattern segment 24f may preferably include 18 cuts, and 18 filars.

The sixth zone 22f may include a plurality of cut pattern segments having the dimensions specified above. The number of cut pattern segments in the sixth zone 22f may be between 1 and 40. Each cut pattern segment of the sixth zone 22f has a segment length between 1.25 mm and 1.75 mm, and may preferably be approximately 1.52 mm. The sixth zone 22f may include a plurality of uncut segments 34, with each uncut segment 34 defining an uncut segment length between 0.05 mm and 0.15 mm, and may preferably be approximately 0.1 mm.

This sixth zone 22f may have an average flexibility between approximately 3.6 gF/mm and approximately 4.32 gF/mm.

Continuing in this example, the catheter body 12 may include a seventh zone 22g located proximal of the sixth zone 22f. The seventh zone 22g may include at least one cut pattern segment 24g having a plurality of circumferentially-spaced cuts 26 having a cut width between 0.01 mm and 0.03 mm, and may preferably be approximately 0.02 mm. The seventh zone 22g may define a plurality of circumferentially-spaced filars 30 having a width between 0.08 mm and 0.12 mm, and may preferably be approximately 0.1 mm.

The plurality of cuts 26 and the plurality of filars 30 may each have a pitch angle α between 19 degrees and 29 degrees, and may preferably be approximately 24 degrees. The cut pattern segment 24g may include between 12 and 24 cuts, and between 12 and 24 filars. The cut pattern segment 24g may preferably include 18 cuts, and 18 filars.

The seventh zone 22g may include a plurality of cut pattern segments having the dimensions specified above. The number of cut pattern segments in the seventh zone 22g may be between 1 and 40. Each cut pattern segment of the seventh zone 22g has a segment length between 1.25 mm and 1.75 mm, and may preferably be approximately 1.52 mm. The seventh zone 22g may include a plurality of uncut segments 34, with each uncut segment 34 defining an uncut segment length between 0.05 mm and 0.15 mm, and may preferably be approximately 0.1 mm.

This seventh zone 22g may have an average flexibility between approximately 4.32 gF/mm and approximately 4.8 gF/mm.

In another example, the catheter body 12 may have an inner diameter between 1.25 mm and 1.75 mm, and outer diameter between 1.5 mm and 1.8 mm, and/or a wall thickness between 0.06 mm and 0.10 mm. Variations of the dimensions and patterns described herein may be incorporated into devices of all applicable inner and outer diameters, including those ranging from 3 French to 34 French.

In this example, the catheter body 12 may include a first zone 22a at or near the distal portion of the medical device 10. The first zone 22a may include at least one cut pattern segment 24a having a plurality of circumferentially-spaced cuts 26 having a cut width between 0.01 mm and 0.03 mm, and may preferably be approximately 0.02 mm. The first zone 22a may define a plurality of circumferentially-spaced filars 30 having a width between 0.04 mm and 0.08 mm, and may preferably be approximately 0.062 mm.

The plurality of cuts 26 and the plurality of filars 30 may each have a pitch angle α between 55 degrees and 65 degrees, and may preferably be approximately 60 degrees. The cut pattern segment 24a may include between 20 and 40 cuts, and between 20 and 40 filars. The cut pattern segment 24a may preferably include 30 cuts, and 30 filars.

The first zone 22a may include a plurality of cut pattern segments having the dimensions specified above. The number of cut pattern segments in the first zone 22a may be between 1 and 40. Each cut pattern segment of the first zone 22a has a segment length between 1.5 mm and 2.5 mm, and may preferably be approximately 1.9 mm. The first zone 22a may include a plurality of uncut segments 34, with each uncut segment 34 defining an uncut segment length between 0.25 mm and 0.5 mm, and may preferably be approximately 0.36 mm.

This first zone 22a may have an average flexibility between approximately 0.1 gF/mm and approximately 45 gF/mm.

Continuing in this example of an exemplary medical device, the catheter body 12 may include a second zone 22b located proximal of the first zone 22a. The second zone 22b may include at least one cut pattern segment 24b having a plurality of circumferentially-spaced cuts 26 having a cut width between 0.01 mm and 0.03 mm, and may preferably be approximately 0.02 mm. The second zone 22b may define a plurality of circumferentially-spaced filars 30 having a width between 0.1 mm and 0.2 mm, and may preferably be approximately 0.14 mm.

The plurality of cuts 26 and the plurality of filars 30 may each have a pitch angle α between 45 degrees and 55 degrees, and may preferably be approximately 50 degrees. The cut pattern segment 24b may include between 16 and 24 cuts, and between 16 and 24 filars. The cut pattern segment 24b may preferably include 20 cuts, and 20 filars.

The second zone 22b may include a plurality of cut pattern segments having the dimensions specified above. The number of cut pattern segments in the second zone 22b may be between 1 and 40. Each cut pattern segment of the second zone 22b has a segment length between 1.5 mm and 2.5 mm, and may preferably be approximately 1.9 mm. The second zone 22b may include a plurality of uncut segments 34, with each uncut segment 34 defining an uncut segment length between 0.25 mm and 0.5 mm, and may preferably be approximately 0.36 mm.

This second zone 22b may have an average flexibility between approximately 45 gF/mm and approximately 154 gF/mm.

Continuing in this example, the catheter body 12 may include a third zone 22c located proximal of the second zone 22b. The third zone 22c may include at least one cut pattern segment 24c having a plurality of circumferentially-spaced cuts 26 having a cut width between 0.01 mm and 0.03 mm, and may preferably be approximately 0.02 mm. The third zone 22c may define a plurality of circumferentially-spaced filars 30 having a width between 0.1 mm and 0.3 mm, and may preferably be approximately 0.17 mm. The plurality of cuts 26 and the plurality of filars 30 may each have a pitch angle α between 35 degrees and 45 degrees, and may preferably be approximately 40 degrees. The cut pattern segment 24c may include between 16 and 24 cuts, and between 16 and 24 filars. The cut pattern segment 24c may preferably include 20 cuts, and 20 filars.

The third zone 22c may include a plurality of cut pattern segments having the dimensions specified above. The number of cut pattern segments in the third zone 22c may be between 1 and 40. Each cut pattern segment of the third zone 22c has a segment length between 1.5 mm and 2.5 mm, and may preferably be approximately 1.9 mm. The third zone 22c may include a plurality of uncut segments 34, with each uncut segment 34 defining an uncut segment length between 0.25 mm and 0.5 mm, and may preferably be approximately 0.36 mm.

This third zone 22c may have an average flexibility between approximately 154 gF/mm and approximately 223 gF/mm.

Continuing in this example of an exemplary medical device, the catheter body 12 may include a fourth zone 22d located proximal of the first zone 22a. The fourth zone 22d may include at least one cut pattern segment 24d having a plurality of circumferentially-spaced cuts 26 having a cut width between 0.015 mm and 0.035 mm, and may preferably be approximately 0.25 mm. The fourth zone 22b may define a plurality of circumferentially-spaced filars 30 having a width between 0.1 mm and 0.3 mm, and may preferably be approximately 0.22 mm.

The plurality of cuts 26 and the plurality of filars 30 may each have a pitch angle α between 25 degrees and 35 degrees, and may preferably be approximately 30 degrees. The cut pattern segment 24d may include between 16 and 24 cuts, and between 16 and 24 filars. The cut pattern segment 24d may preferably include 20 cuts, and 20 filars.

The fourth zone 22d may include a plurality of cut pattern segments having the dimensions specified above. The number of cut pattern segments in the fourth zone 22d may be between 1 and 40. Each cut pattern segment of the fourth zone 22d has a segment length between 1.5 mm and 2.5 mm, and may preferably be approximately 2 mm. The fourth zone 22d may include a plurality of uncut segments 34, with each uncut segment 34 defining an uncut segment length between 0.25 mm and 0.5 mm, and may preferably be approximately 0.36 mm.

This fourth zone 22d may have an average flexibility between approximately 223 gF/mm and approximately 245 gF/mm.

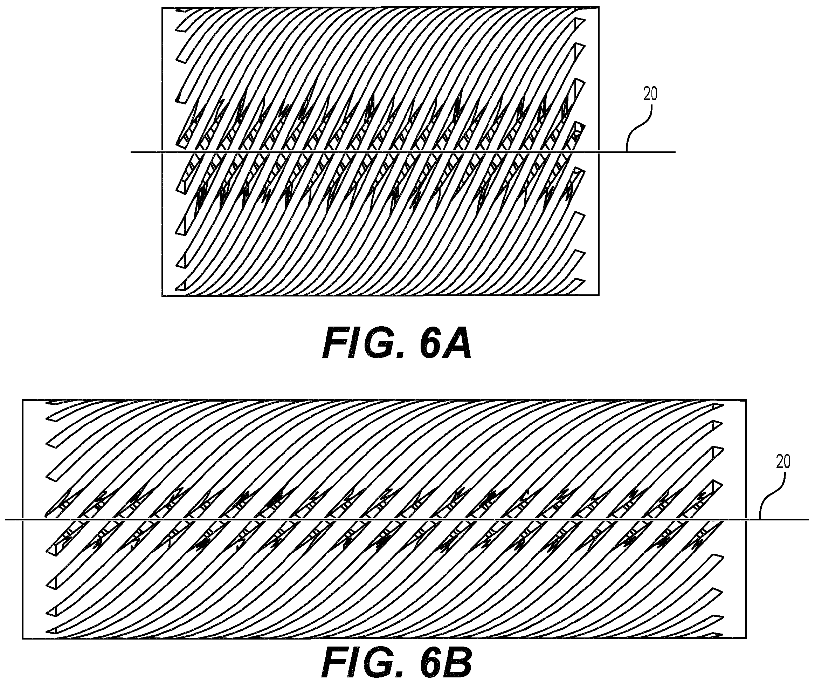

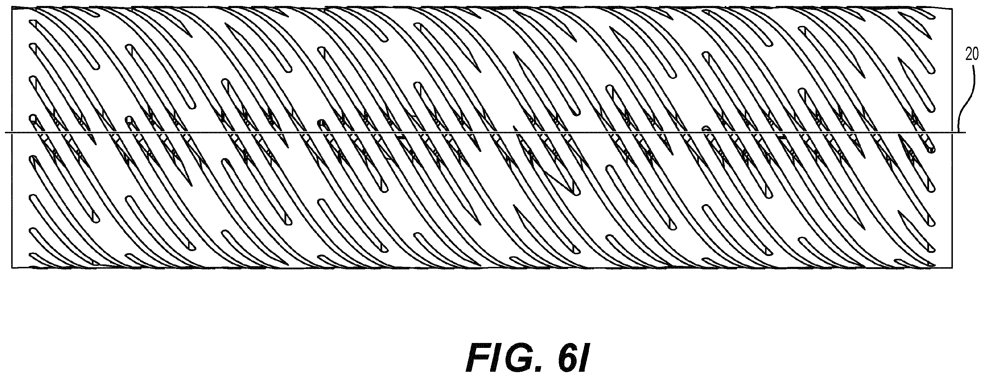

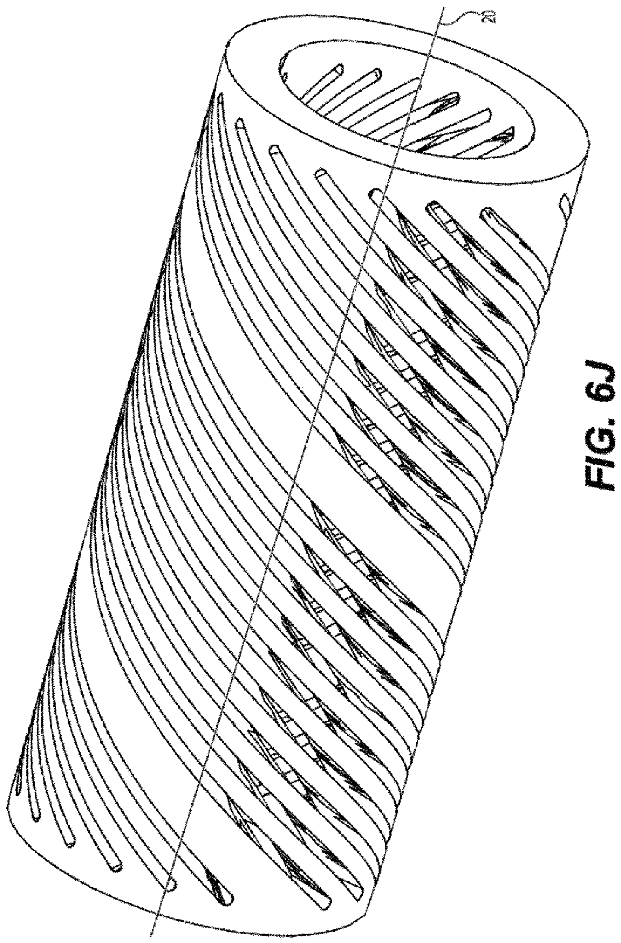

FIGS. 6A-6D show additional examples of cut pattern segments for the catheter body 12. For example, FIG. 6A shows a cut pattern segment having a plurality of circumferentially-spaced cuts 26 having a cut width between 0.02 mm and 0.04 mm, and may preferably be approximately 0.03 mm, and a plurality of circumferentially-spaced filars 30 having a width between 0.05 mm and 0.07 mm, and may preferably be approximately 0.058 mm. The plurality of cuts 26 and the plurality of filars 30 may each have a pitch angle α between 55 degrees and 65 degrees, and may preferably be approximately 60 degrees. The cut pattern segment may include between 25 and 35 cuts, and between 25 and 35 filars, and preferably include 30 cuts and 30 filars. The segment may have a length between 2 mm and 3 mm.

FIG. 6B shows a cut pattern segment having a plurality of circumferentially-spaced cuts 26 having a cut width between 0.02 mm and 0.04 mm, and may preferably be approximately 0.03 mm, and a plurality of circumferentially-spaced filars 30 having a width between 0.07 mm and 0.09 mm, and may preferably be approximately 0.08 mm. The plurality of cuts 26 and the plurality of filars 30 may each have a pitch angle α between 45 degrees and 55 degrees, and may preferably be approximately 50 degrees. The cut pattern segment may include between 25 and 35 cuts, and between 25 and 35 filars, and preferably include 30 cuts and 30 filars. The segment may have a length between 4 mm and 6mm.

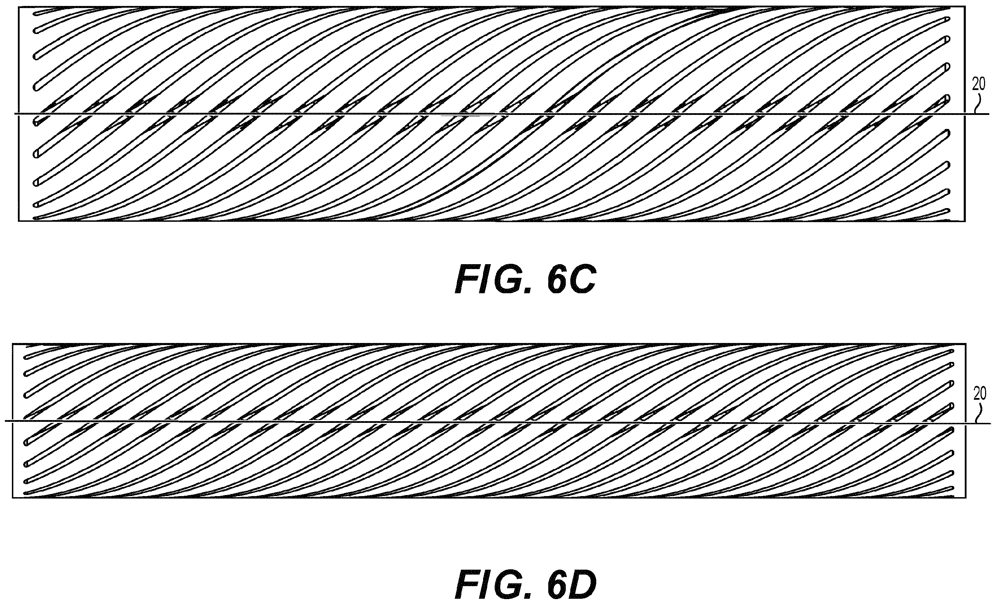

FIG. 6C shows a cut pattern segment having a plurality of circumferentially-spaced cuts 26 having a cut width between 0.02 mm and 0.04 mm, and may preferably be approximately 0.03 mm, and a plurality of circumferentially-spaced filars 30 having a width between 0.08 mm and 0.12 mm, and may preferably be approximately 0.1 mm. The plurality of cuts 26 and the plurality of filars 30 may each have a pitch angle α between 35 degrees and 45 degrees, and may preferably be approximately 40 degrees. The cut pattern segment may include between 25 and 35 cuts, and between 25 and 35 filars, and preferably include 30 cuts and 30 filars. The segment may have a length between 6 mm and 8 mm.

FIG. 6D shows a cut pattern segment having a plurality of circumferentially-spaced cuts 26 having a cut width between 0.02 mm and 0.04 mm, and may preferably be approximately 0.03 mm, and a plurality of circumferentially-spaced filars 30 having a width between 0.10 mm and 0.15 mm, and may preferably be approximately 0.12 mm. The plurality of cuts 26 and the plurality of filars 30 may each have a pitch angle α between 25 degrees and 35 degrees, and may preferably be approximately 30 degrees. The cut pattern segment may include between 25 and 35 cuts, and between 25 and 35 filars, and preferably include 30 cuts and 30 filars. The segment may have a length between 8 mm and 12 mm.

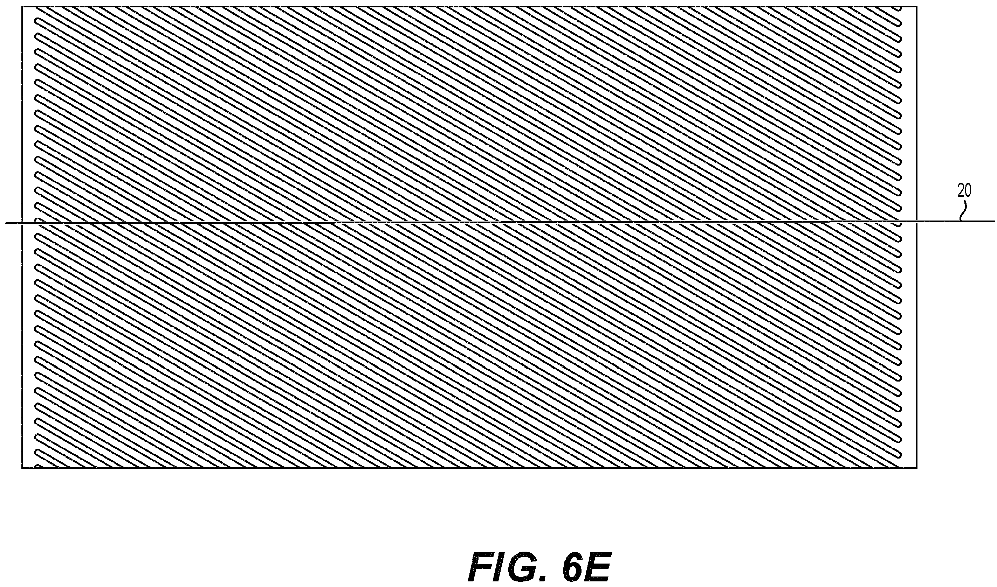

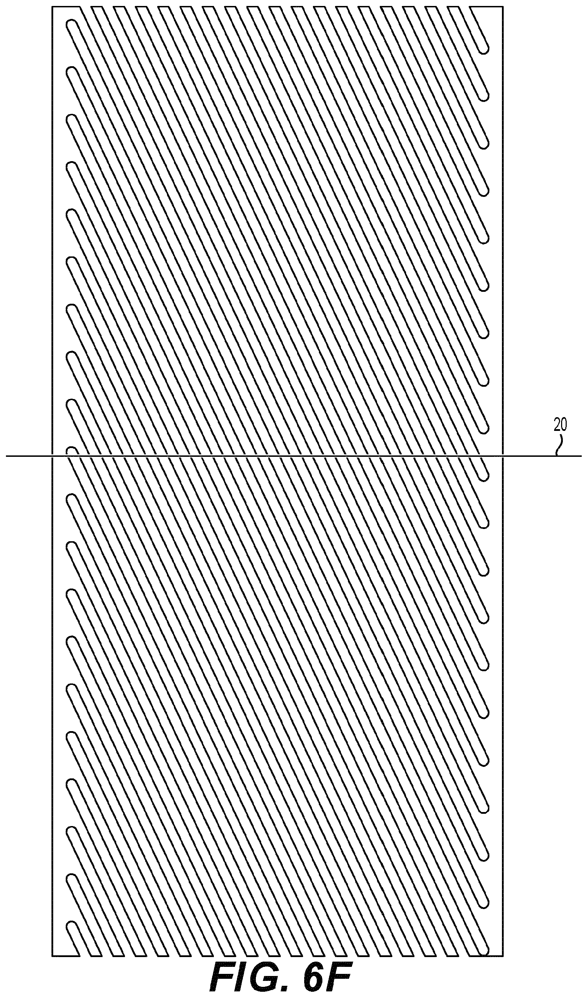

FIGS. 6E and 6F show examples of flattened images of cut pattern segments disclosed herein, illustrating the significant variations in pitch angle and orientation of cut pattern segments that may be implemented along the length of the medical device 10 to provide the desired characteristics disclosed herein.

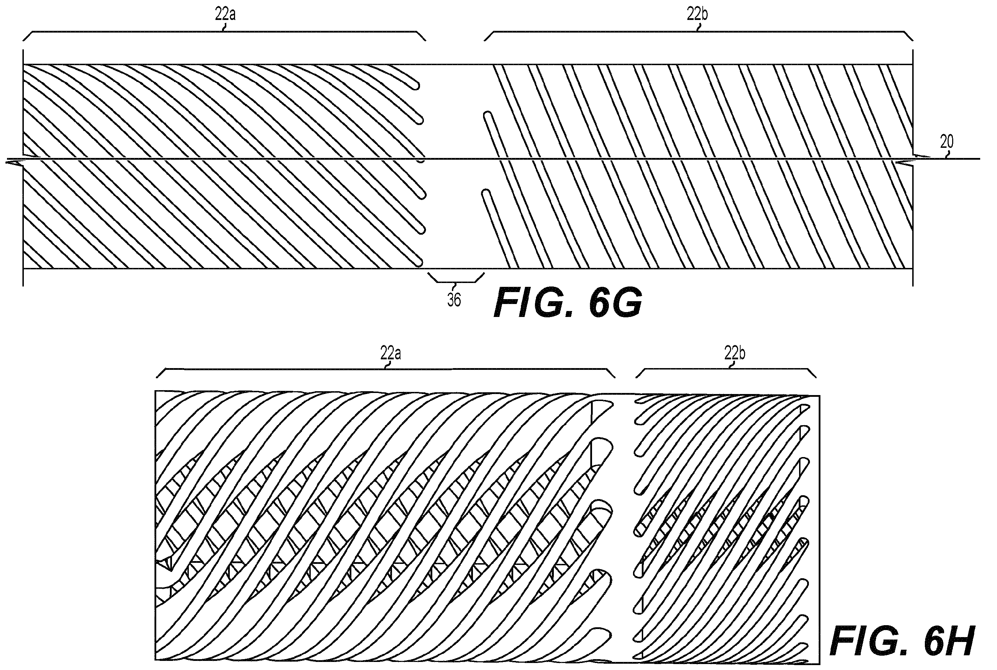

FIGS. 6G and 6H show additional examples of cut pattern segments having significant variations in pitch angle and orientation of cut pattern segments that may be implemented along the length of the medical device 10. For example, FIGS. 6G and 6H illustrate examples where the pitch angle of the cuts and filars shifts dramatically between a first zone 22a and an adjacent second zone 22b to enable the catheter frame to be shape set into a curve from a straight position without compromising the cut pattern or lumen concentricity.

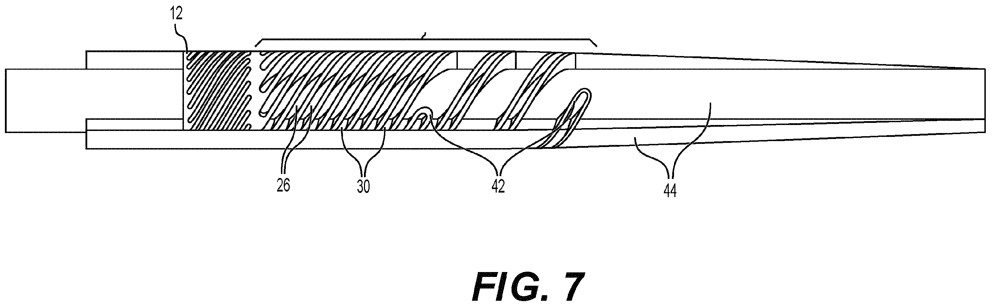

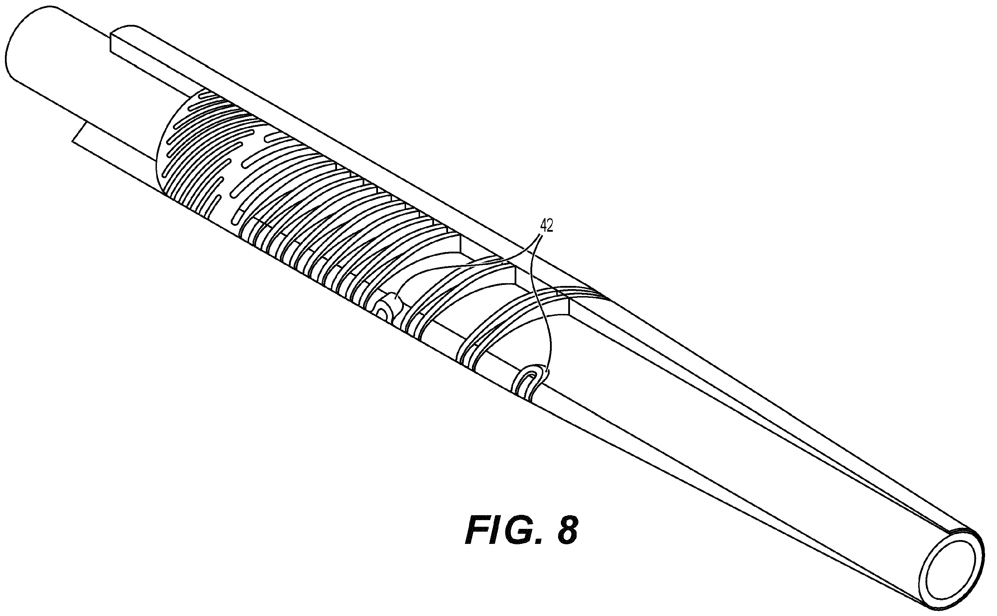

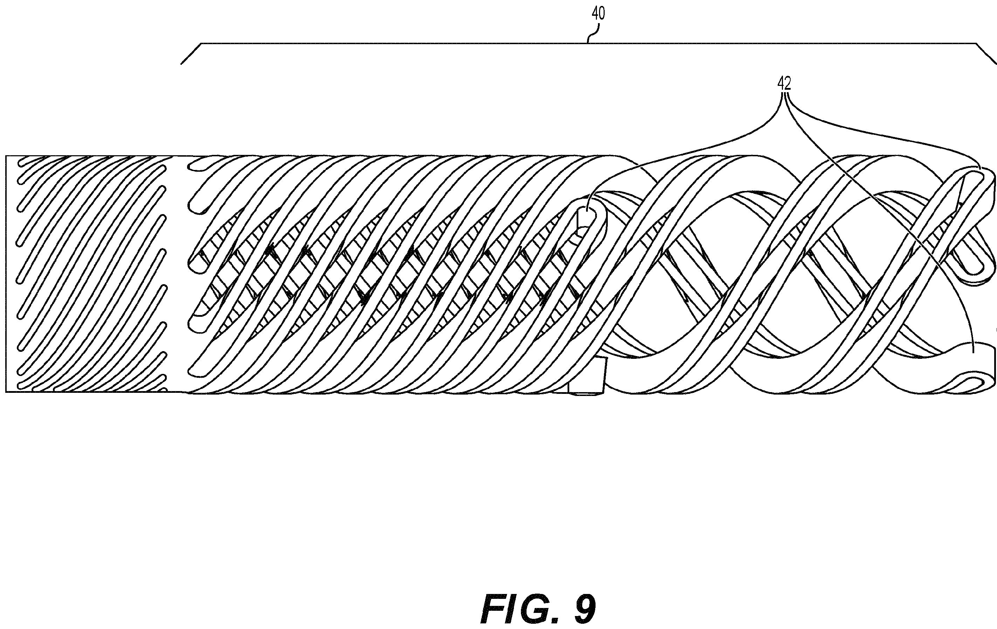

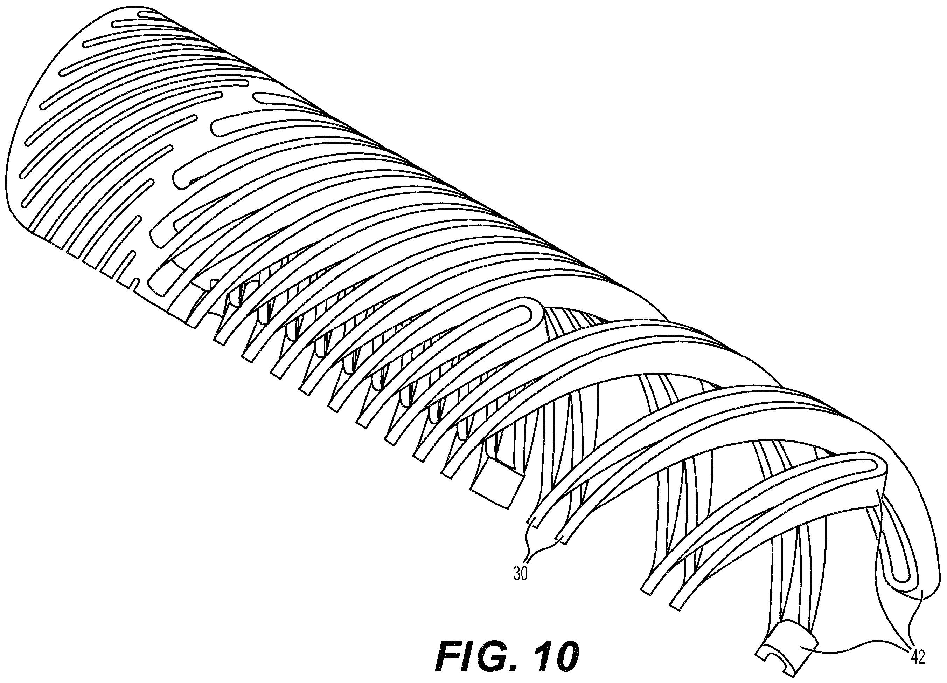

Now referring to FIGS. 7-12, the catheter body may include a distal tip segment 40 at or near the distal-most end of the catheter body 12. The distal tip segment 40 may include one or more cut pattern segments having a plurality of cuts 26 and filars 30 as described herein. Individual pairs of filars 30 within a cut pattern segment of the distal tip segment 40 may be coupled, joined, or otherwise formed together to terminate at a filar tip 42, thus eliminating a "single wire tip" sharp leading edge. The filar tip 42 may be rounded or otherwise have an atraumatic profile to avoid penetrating one or more linings or protective layers of the medical device 10. The paired filars 30 thus form a plurality of helical segments extending around the longitudinal axis 20 of the catheter body 12.

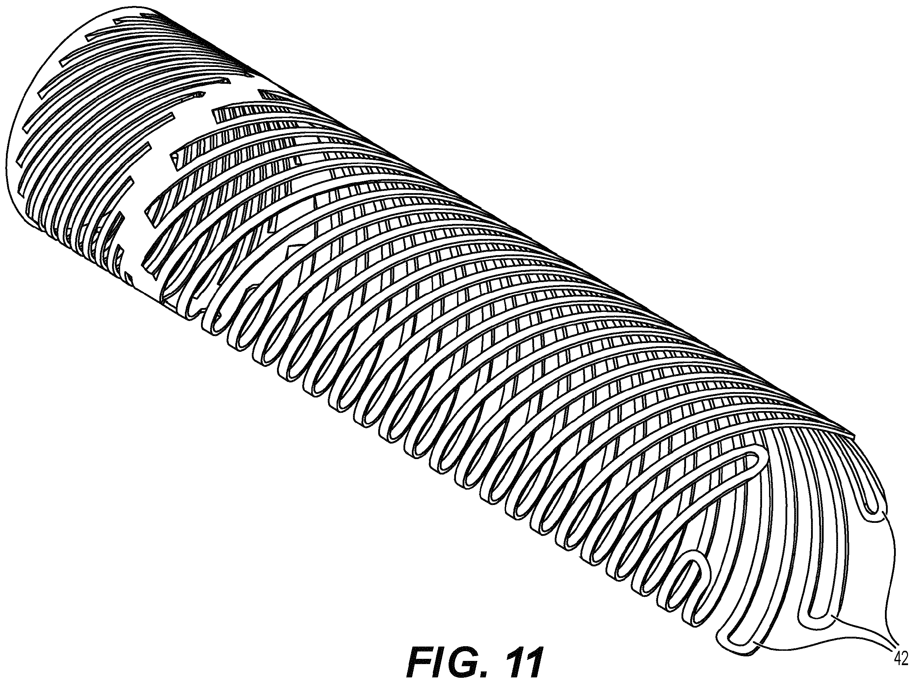

The pairs of filars/helical segments may terminate at different lengths along the longitudinal axis of the catheter body 12, examples of which are shown in FIGS. 7-10. Alternatively, the pairs of filars/helical segments of a distal tip segment 40 may terminate at substantially coplanar or single longitudinal position along the catheter body 12, as shown in FIGS. 11-12.

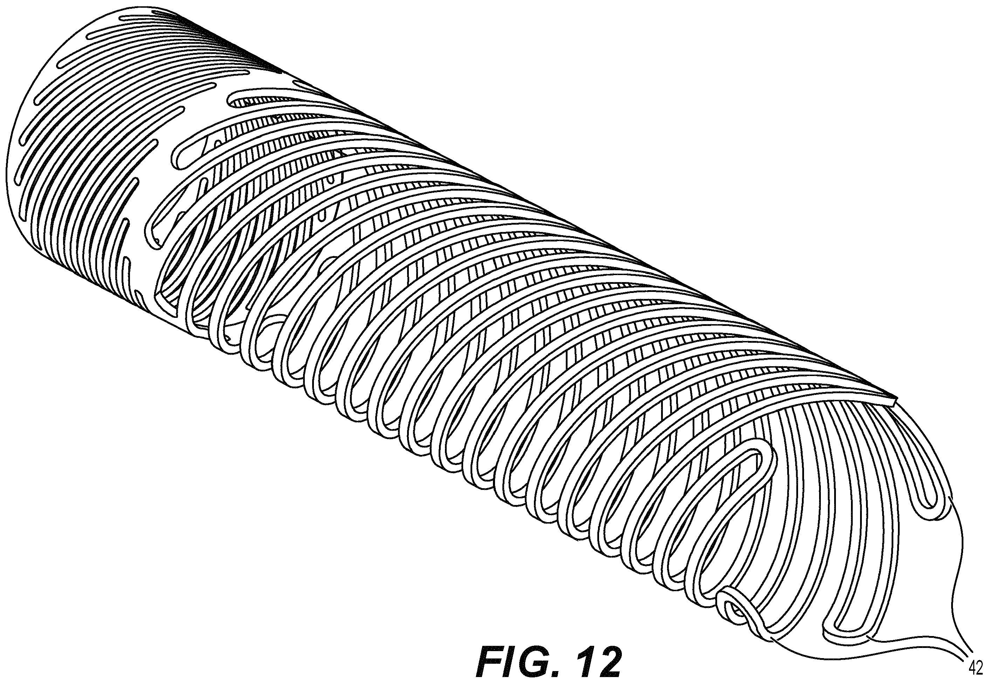

The direction of the pitch of the filars 30 within the distal tip segment 40 may vary, and the quantity of the filars 30 within the circumference of a particular distal tip segment 40 may also vary to provide the desired characteristics. For example, the distal tip segment shown in FIGS. 7-10 includes 12 filars constituting 6 paired helical segments with staggered termination points along the length of the distal tip segment 40. Such staggard termination will produce a graduation of axial stiffness and enable a reduction in the tip compliance from the catheter segmented body into the tip paired helical segments 40 and molded tip body 44. FIG. 11 illustrates an example of a distal tip segment 40 having 12 filars constituting 6 paired helical segments that jointly terminate at a single position along the length of the distal tip segment 40. FIG. 12 illustrates an example of a distal tip segment 40 having 10 filars constituting 5 paired helical segments that jointly terminate at a single position along the length of the distal tip segment 40.

In one example, distal tip segment 40 may include at least one cut pattern segment having a plurality of circumferentially-spaced cuts 26 having a cut width between 0.025 mm and 0.1 mm, and a plurality of circumferentially-spaced filars 30 having a width between 0.02 mm and 0.05 mm. The plurality of cuts 26 and the plurality of filars 30 may each have a pitch angle α between 50 degrees and 65 degrees. The distal tip segment 40 may include between 16 and 24 cuts, and between 16 and 24 filars. The distal tip segment 40 may have a segment length between 2 mm and 6 mm.

In another example, distal tip segment 40 may include at least one cut pattern segment having a plurality of circumferentially-spaced cuts 26 having a cut width between 0.07 mm and 0.12 mm, and a plurality of circumferentially-spaced filars 30 having a width between 0.02 mm and 0.1 mm. The plurality of cuts 26 and the plurality of filars 30 may each have a pitch angle α between 55 degrees and 65 degrees. The distal tip segment 40 may include between 16 and 24 cuts, and between 16 and 24 filars. The distal tip segment 40 may have a segment length between 1 mm and 4 mm.

In another example, distal tip segment 40 may include at least one cut pattern segment having a plurality of circumferentially-spaced cuts 26 having a cut width between 0.05 mm and 0.10 mm, and a plurality of circumferentially-spaced filars 30 having a width between 0.02 mm and 0.05 mm. The plurality of cuts 26 and the plurality of filars 30 may each have a pitch angle α between 55 degrees and 65 degrees. The distal tip segment 40 may include between 16 and 24 cuts, and between 16 and 24 filars. The distal tip segment 40 may have a segment length between 1 mm and 4 mm.

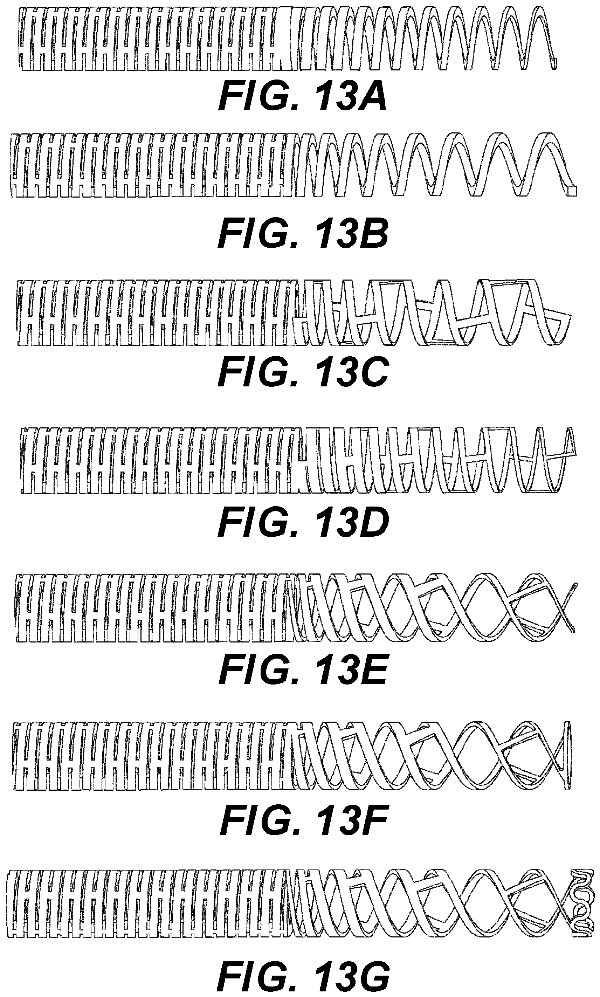

Additional examples of the distal tip segment 40 are shown in FIGS. 13A-G, having varying static or progressive cut widths, pitch angles, filar widths and/or axial struts therebetween.

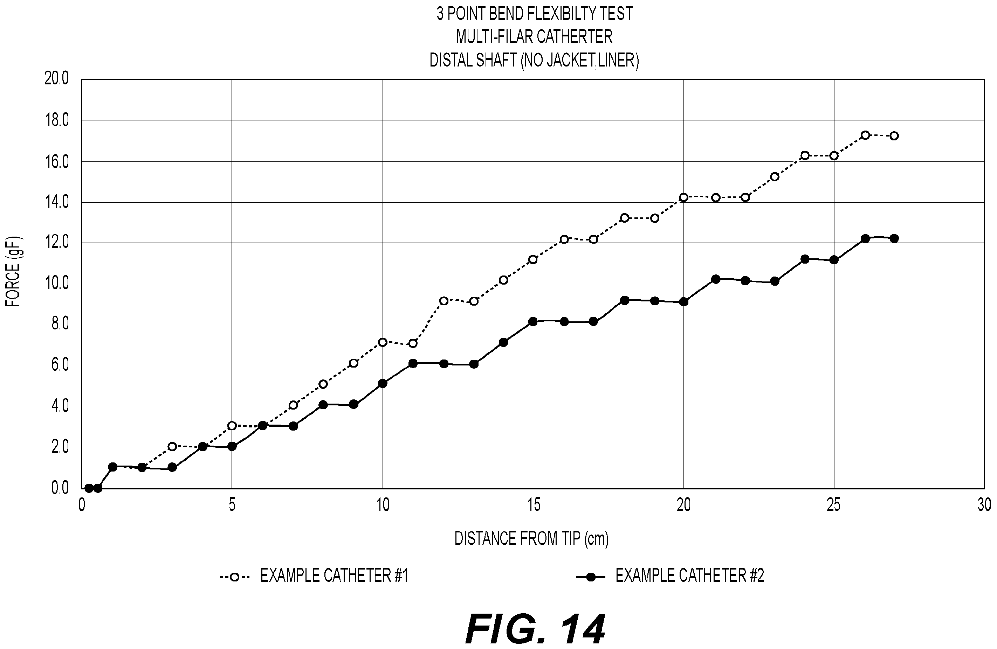

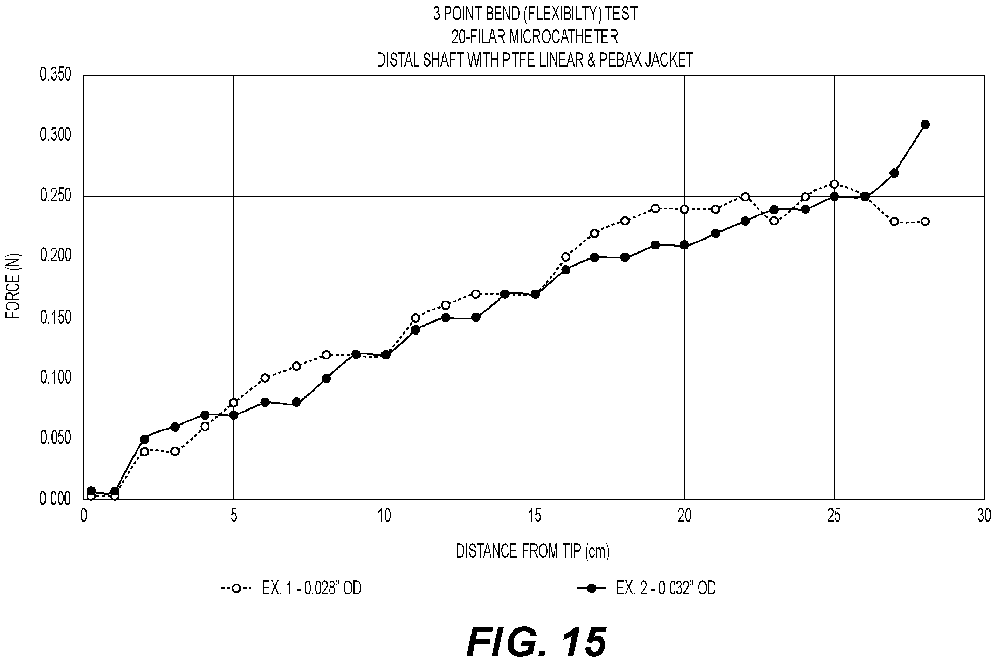

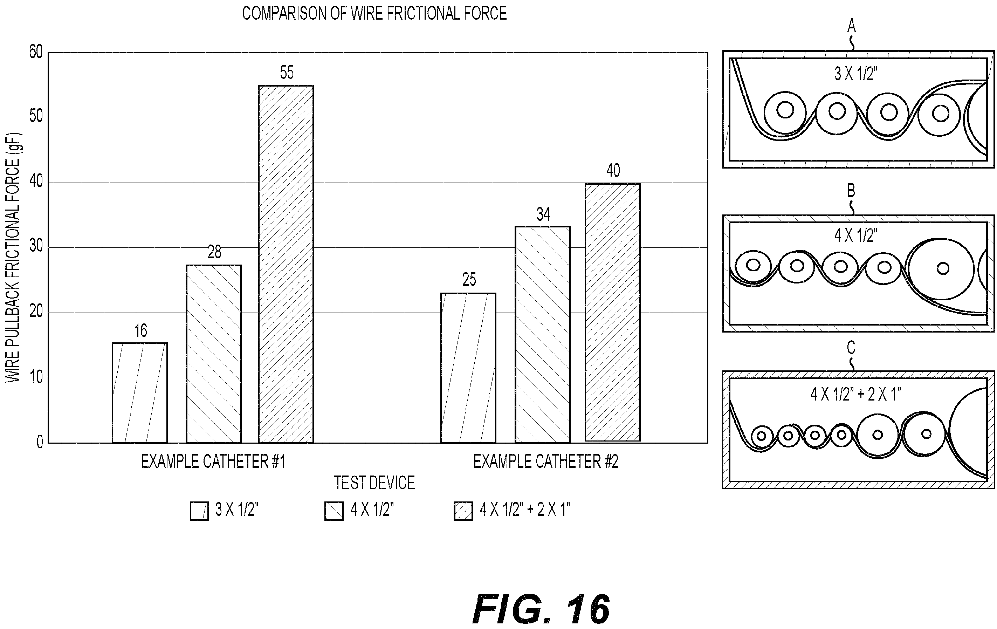

Now referring to FIGS. 14-17, graphs of recorded test results for examples of the medical device 10 and components thereof described herein are shown. FIG. 14 shows data regarding a 3-point bend test along the length of a catheter body having a definable progressive flexibility along the length of the catheter body in a proximal-to-distal direction. FIG. 15 similarly shows data regarding a 3-point bend test along the length of a catheter body with a polymer liner and jacket, the catheter body assembly having a definable progressive flexibility along the length of the catheter body in a proximal-to-distal direction. FIG. 16 shows guidewire frictional force data when a guidewire is removed from within a catheter body constructed as disclosed herein under varying distal track contours simulating vascular anatomy, demonstrating ease of use in over-the-wire applications. The different simulated paths for the curvature of the catheter under which the frictional force was measured is shown in pictures of the test fixture labeled 'A', 'B', and 'C'.

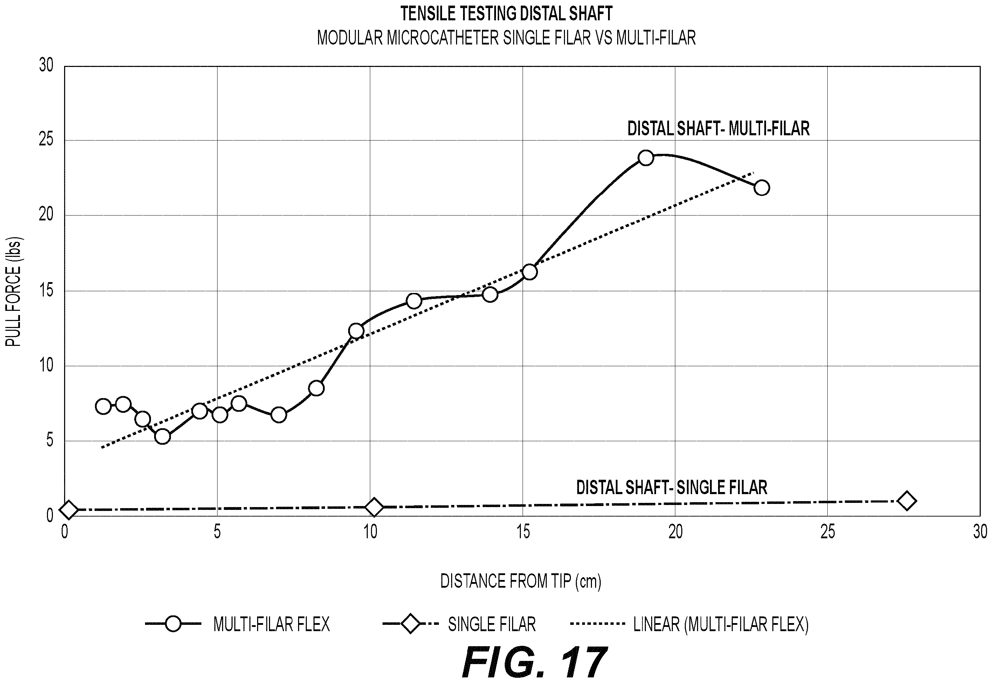

FIG. 17 illustrates a graph of axial pull force testing on an example of a catheter body constructed as disclosed herein (e.g., "Multi-Filar") versus a single filar construction. As shown by the graph, the multi-filar construction provides increased axial pull strength compared to the single filar construct, and the multi-filar construction provides such improved axial pull strength while also maintaining a high degree of flexibility as shown in the other tables and graphs disclosed herein.

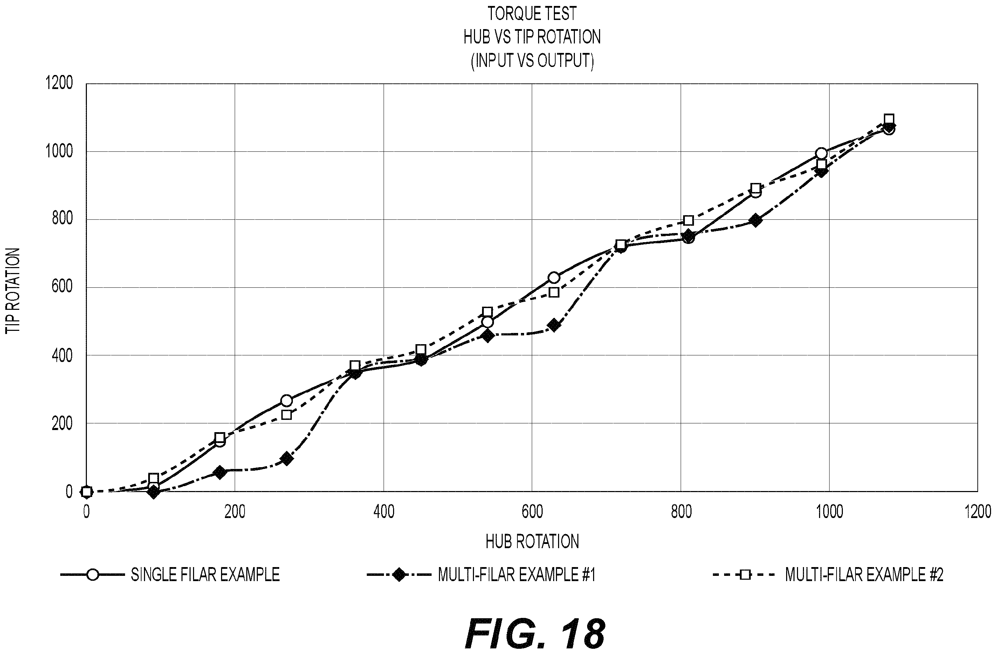

FIG. 18 illustrates a graph of torque transmission testing on an example of a catheter body constructed as disclosed herein (e.g., "Multi-Filar") versus a single filar construction. As shown by the graph, the multi-filar construction provides equivalent torque response in both clockwise and counter clockwise transmission compared to a single filar construct, and the multi-filar construction provides equivalent torque transmission while also maintaining a high degree of flexibility as shown in the other tables and graphs disclosed herein.

The examples and associated dimensions of the medical devices 10 described above may be modified for different applications to conform to and/or adapt for use in different anatomical structures and/or for different patients. One or more portions of the device 10 may be radiopaque and/or include radiopaque markers to aid in medical imaging of the device during a procedure. Radiopaque features may be achieved, for example, through the use of marker bands, plating, the inclusion or infusion of tantalum, platinum, gold, tungsten, bismuth, and/or barium sulphate into or onto one or more components of the device. The device 10 may also include one or more polymer liners and outer jackets as well as coating(s) 44 on surfaces thereof along the length of the device. A filled polymer with one or more of these radiopaque materials may be melted into the distal tip pattern to enable a continuous transition of flexibility compliance from the catheter shaft to the distal polymer tip end.

It will be appreciated by persons skilled in the art that the present disclosure is not limited to what has been particularly shown and described herein above. In addition, unless mention was made above to the contrary, it should be noted that all of the accompanying drawings are not to scale. Of note, the system components have been represented where appropriate by conventional symbols in the drawings, showing only those specific details that are pertinent to understanding the embodiments of the present disclosure so as not to obscure the disclosure with details that will be readily apparent to those of ordinary skill in the art having the benefit of the description herein. Moreover, while certain embodiments or figures described herein may illustrate features not expressly indicated on other figures or embodiments, it is understood that the features and components of the examples disclosed herein are not necessarily exclusive of each other and may be included in a variety of different combinations or configurations without departing from the scope defined by the claims. A variety of modifications and variations are possible in light of the above teachings without departing from the scope of the disclosure, which is limited only by claims.

## Claims

1. A catheter (10), comprising:
a catheter tube (12) defining:
a proximal end (14);
a distal end (16);
a longitudinal axis (20);
a lumen (18) therethrough; and
a first zone (22) having a first cut pattern segment (24) therein,
wherein the first cut pattern segment (24) defines a plurality of spiral cuts (26) spaced around a circumference of the tube (12),
wherein each of the plurality of spiral cuts (26) defines a width (28) between 0.015 mm and 0.040 mm,
wherein each of the plurality of spiral cuts (26) is spaced from an adjacent cut (26) by a filar (30) having a width (32) between 0.015 mm and 0.125 mm, and
wherein each of the plurality of spiral cuts (26) defines a pitch angle (α) with the longitudinal axis (20) between 30 degrees and 70 degrees.

2. The catheter (10) of claim 1, wherein the first zone (22) has a stiffness between approximately 0.1 gF/mm and approximately 0.75 gF/mm.

3. The catheter (10) of claims 1 or 2, wherein the plurality of spiral cuts (26) includes between 10 and 40 cuts (26).

4. The catheter (10) of any one of claims 1 to 3, wherein the first zone (22) has a length between 1 mm and 100 mm.

5. The catheter (10) of any one of claims 1 to 4, wherein the catheter tube (12) is constructed from a nitinol and has a wall thickness between approximately 0.0254 mm and approximately 0.254 mm.

6. The catheter (10) of any one of claims 1 to 5, wherein the catheter tube (12) is constructed from an inconel and has a wall thickness between approximately 0.0254 mm and approximately 0.254 mm.

7. The catheter (10) of any one of claims 1 to 6, wherein the catheter tube (12) is constructed from stainless steel and has a wall thickness between approximately 0.0254 mm and approximately 0.254 mm.

8. The catheter (10) of any one of claims 1 to 7, wherein the distal end (16) includes a tip segment having a plurality of helical segments extending around the longitudinal axis (20).

9. The catheter (10) of claim 8, wherein each helical segment includes two substantially parallel filars (30) connected at distal ends thereof.

10. The catheter (10) of claim 9, wherein the plurality of helical segments includes at least 2 helical segments.

11. The catheter (10) of any one of claims 1 to 10, further comprising a polymer liner disposed within the lumen (18) and/or a polymer jacket disposed around the outer diameter of the cut tube (12).

12. The catheter (10) of any one of claims 1 to 11, wherein the catheter tube (12) further comprises a second zone (22) having a second cut pattern segment (24) therein,
wherein the second cut pattern segment (24) defines a plurality of cuts (26) spaced around a circumference of the tube (12),
wherein each of the plurality of cuts (26) defines a width (28) between 0.015 mm and 0.04 mm,
wherein each of the plurality of cuts (26) is spaced from an adjacent cut (26) by a filar (30) having a width (32) between 0.015 mm and 0.125 mm, and
wherein each of the plurality of cuts (26) defines a pitch angle (α) with the longitudinal axis (20) between 45 degrees and 55 degrees.

13. The catheter (10) of claim 12, wherein a) the second zone (22) is located proximal of the first zone (22), b) the second zone (22) has a stiffness between approximately 0.75 gF/mm and approximately 1.25 gF/mm, c) the plurality of cuts (26) of the second cut pattern segment (24) includes between 1 and 40 cuts and/or d) the second zone (22) has a length between 1 mm and 100 mm.

## Patentansprüche

1. Katheter (10), umfassend:
eine Katheterröhre (12), die Folgendes definiert:
ein proximales Ende (14);
ein distales Ende (16);
eine Längsachse (20);
ein Lumen (18) dadurch; und
eine erste Zone (22), die ein erstes Schnittmustersegment (24) darin aufweist,
wobei das erste Schnittmustersegment (24) eine Vielzahl von spiralförmigen Schnitten (26) definiert, die um einen Umfang der Röhre (12) herum beabstandet sind,
wobei jeder aus der Vielzahl von spiralförmigen Schnitten (26) eine Breite (28) zwischen 0,015 mm und 0,040 mm definiert,
wobei jeder aus der Vielzahl von spiralförmigen Schnitten (26) von einem benachbarten Schnitt (26) durch eine Faser (30) mit einer Breite (32) zwischen 0,015 mm und 0,125 mm beabstandet ist und
wobei jeder aus der Vielzahl von spiralförmigen Schnitten (26) einen Steigungswinkel (α) mit der Längsachse (20) zwischen 30 Grad und 70 Grad definiert.

2. Katheter (10) nach Anspruch 1, wobei die erste Zone (22) eine Steifigkeit zwischen etwa 0,1 gF/mm und etwa 0,75 gF/mm aufweist.

3. Katheter (10) nach Anspruch 1 oder 2, wobei die Vielzahl von spiralförmigen Schnitten (26) zwischen 10 und 40 Schnitte (26) enthält.

4. Katheter (10) nach einem der Ansprüche 1 bis 3, wobei die erste Zone (22) eine Länge zwischen 1 mm und 100 mm aufweist.

5. Katheter (10) nach einem der Ansprüche 1 bis 4, wobei die Katheterröhre (12) aus einem Nitinol aufgebaut ist und eine Wandstärke zwischen etwa 0,0254 mm und etwa 0,254 mm aufweist.

6. Katheter (10) nach einem der Ansprüche 1 bis 5, wobei die Katheterröhre (12) aus einem Inconel aufgebaut ist und eine Wandstärke zwischen etwa 0,0254 mm und etwa 0,254 mm aufweist.

7. Katheter (10) nach einem der Ansprüche 1 bis 6, wobei die Katheterröhre (12) aus rostfreiem Stahl aufgebaut ist und eine Wandstärke zwischen etwa 0,0254 mm und etwa 0,254 mm aufweist.

8. Katheter (10) nach einem der Ansprüche 1 bis 7, wobei das distale Ende (16) ein Spitzensegment enthält, das eine Vielzahl von schraubenförmigen Segmenten aufweist, die sich um die Längsachse (20) erstrecken.

9. Katheter (10) nach Anspruch 8, wobei jedes schraubenförmige Segment zwei im Wesentlichen parallele Fasern (30) enthält, die an distalen Enden davon verbunden sind.

10. Katheter (10) nach Anspruch 9, wobei die Vielzahl von schraubenförmigen Segmenten mindestens 2 schraubenförmige Segmente enthält.

11. Katheter (10) nach einem der Ansprüche 1 bis 10, ferner umfassend eine Polymerauskleidung, die innerhalb des Lumens (18) angeordnet ist, und/oder eine Polymerummantelung, die um den Außendurchmesser der geschnittenen Röhre (12) herum angeordnet ist.

12. Katheter (10) nach einem der Ansprüche 1 bis 11, wobei die Katheterröhre (12) ferner eine zweite Zone (22) mit einem zweiten Schnittmustersegment (24) darin umfasst,
wobei das zweite Schnittmustersegment (24) eine Vielzahl von Schnitten (26) definiert, die um einen Umfang der Röhre (12) herum beabstandet sind,
wobei jeder aus der Vielzahl von Schnitten (26) eine Breite (28) zwischen 0,015 mm und 0,04 mm definiert,
wobei jeder aus der Vielzahl von Schnitten (26) von einem benachbarten Schnitt (26) durch eine Faser (30) mit einer Breite (32) zwischen 0,015 mm und 0,125 mm beabstandet ist und
wobei jeder aus der Vielzahl von Schnitten (26) einen Steigungswinkel (α) mit der Längsachse (20) zwischen 45 Grad und 55 Grad definiert.

13. Katheter (10) nach Anspruch 12, wobei a) sich die zweite Zone (22) proximal der ersten Zone (22) befindet, b) die zweite Zone (22) eine Steifigkeit zwischen etwa 0,75 gF/mm und etwa 1,25 gF/mm aufweist, c) die Vielzahl von Schnitten (26) des zweiten Schnittmustersegments (24) zwischen 1 und 40 Schnitte enthält und/oder d) die zweite Zone (22) eine Länge zwischen 1 mm und 100 mm aufweist.

## Revendications

1. Cathéter (10), comprenant :
un tube cathéter (12) définissant :
une extrémité proximale (14) ;
une extrémité distale (16) ;
un axe longitudinal (20) ;
une lumière (18) à travers celui-ci ; et
une première zone (22) comportant un premier segment de motif de coupe (24) dans celle-ci,
dans lequel le premier segment de motif de coupe (24) définit une pluralité de coupes en spirale (26) espacées autour d'une circonférence du tube (12),
dans lequel chacune de la pluralité de coupes en spirale (26) définit une largeur (28) comprise entre 0,015 mm et 0,040 mm,
dans lequel chacune de la pluralité de coupes en spirale (26) est espacée d'une coupe adjacente (26) par un fil (30) ayant une largeur (32) comprise entre 0,015 mm et 0,125 mm, et
dans lequel chacune de la pluralité de coupes en spirale (26) définit un angle de pas (α) avec l'axe longitudinal (20) compris entre 30 degrés et 70 degrés.

2. Cathéter (10) selon la revendication 1, dans lequel la première zone (22) présente une rigidité comprise entre approximativement 0,1 gF/mm et approximativement 0,75 gF/mm.

3. Cathéter (10) selon la revendication 1 ou 2, dans lequel la pluralité de coupes en spirale (26) comprend entre 10 et 40 coupes (26).

4. Cathéter (10) selon l'une quelconque des revendications 1 à 3, dans lequel la première zone (22) présente une longueur comprise entre 1 mm et 100 mm.

5. Cathéter (10) selon l'une quelconque des revendications 1 à 4, dans lequel le tube de cathéter (12) est construit à partir d'un nitinol et présente une épaisseur de paroi comprise entre environ 0,0254 mm et environ 0,254 mm.

6. Cathéter (10) selon l'une quelconque des revendications 1 à 5, dans lequel le tube de cathéter (12) est construit à partir d'un inconel et présente une épaisseur de paroi comprise entre environ 0,0254 mm et environ 0,254 mm.

7. Cathéter (10) selon l'une quelconque des revendications 1 à 6, dans lequel le tube de cathéter (12) est construit à partir d'acier inoxydable et présente une épaisseur de paroi comprise entre environ 0,0254 mm et environ 0,254 mm.

8. Cathéter (10) selon l'une quelconque des revendications 1 à 7, dans lequel l'extrémité distale (16) comprend un segment d'extrémité ayant une pluralité de segments hélicoïdaux s'étendant autour de l'axe longitudinal (20).

9. Cathéter (10) selon la revendication 8, dans lequel chaque segment hélicoïdal comprend deux filaires sensiblement parallèles (30) reliés à leurs extrémités distales.

10. Cathéter (10) selon la revendication 9, dans lequel la pluralité de segments hélicoïdaux comprend au moins 2 segments hélicoïdaux.

11. Cathéter (10) selon l'une quelconque des revendications 1 à 10, comprenant en outre un revêtement polymère disposé à l'intérieur de la lumière (18) et/ou une gaine polymère disposée autour du diamètre extérieur du tube coupé (12).

12. Cathéter (10) selon l'une quelconque des revendications 1 à 11, dans lequel le tube de cathéter (12) comprend en outre une seconde zone (22) comportant un second segment de motif de coupe (24) dans celle-ci,
dans lequel le premier segment de motif de coupe (24) définit une pluralité de coupes en spirale (26) espacées autour d'une circonférence du tube (12),
dans lequel chacune de la pluralité de coupes en spirale (26) définit une largeur (28) comprise entre 0,015 mm et 0,04 mm,
dans lequel chacune de la pluralité de coupes en spirale (26) est espacée d'une coupe adjacente (26) par un fil (30) ayant une largeur (32) comprise entre 0,015 mm et 0,125 mm, et
dans lequel chacune de la pluralité de coupes (26) définit un angle de pas (α) avec l'axe longitudinal (20) compris entre 45 degrés et 55 degrés.

13. Cathéter (10) selon la revendication 12, dans lequel a) la seconde zone (22) est située à proximité de la première zone (22), b) la seconde zone (22) présente une rigidité comprise entre approximativement 0,75 gF/mm et approximativement 1,25 gF/mm, c) la pluralité de coupes (26) du second segment de motif de coupe (24) comprend entre 1 et 40 coupes et/ou d) la seconde zone (22) présente une longueur comprise entre 1 mm et 100 mm.
